# EUROPEAN PATENT APPLICATION

(11) **EP 4 019 550 A1**
(43) Date of publication of application: **29.06.2022**
(21) Application number: 20854633.3
(22) Date of filing: 06.07.2020
(51) Int. Cl.: C07K 16/28, C12N 15/13, G01N 33/68, A61K 39/395, A61P 35/00, A61P 37/00, C07K 16/46, C07K 19/00, C12N 15/62

(54) **ANTI-PD-L1 SINGLE DOMAIN ANTIBODIES**

(30) Priority: 22.08.2019 CN 201910777959
(71) Applicant: Zhejiang Doer Biologics Co., Ltd., Hangzhou, Zhejiang 310018 (CN)
(72) Inventor: YAO, Gaofeng, Hangzhou, Zhejiang 310018 (CN); ZHOU, Zhenxing, Hangzhou, Zhejiang 310018 (CN); CHEN, Yonglu, Hangzhou, Zhejiang 310018 (CN); YANG, Zhiyu, Hangzhou, Zhejiang 310018 (CN); DONG, Jiali, Hangzhou, Zhejiang 310018 (CN); WEN, Xiaofang, Hangzhou, Zhejiang 310018 (CN); HUANG, Yanshan, Hangzhou, Zhejiang 310018 (CN)
(74) Representative: Canzler & Bergmeier Patentanwälte Partnerschaft mbB
(86) International application number: PCT/CN2020/100462
(87) International publication number: WO 2021/031716

(57) **Abstract**

Provided are a class of specific anti-PD-L1 single domain antibodies that block the interaction of PD-L1/PD-1, and humanized antibodies, fusion proteins, pharmaceutical compositions prepared on the basis of said single-domain antibodies, and use thereof.

SEQUENCE LISTING

<110>  ZHEJIANG DOER BIOLOGICS CO., LTD.

<120>  ANTI-PD-L1 SINGLE-DOMAIN ANTIBODIES

<130>  210889&210818&210819

<160>  68

<170>  PatentIn version 3.5

<210>  1
<211>  25
<212>  PRT
<213>  Mus musculus

<400>  1

Gln Leu Gln Leu Val Glu Ser Gly Gly Asp Leu Val Gln Pro Gly Gly
1               5                   10                  15

Ser Leu Arg Leu Ser Cys Ala Ala Ser
            20                  25

<210>  2
<211>  25
<212>  PRT
<213>  Mus musculus

<400>  2

Gln Leu Gln Leu Val Glu Ser Gly Gly Gly Leu Val Gln Ala Gly Gly
1               5                   10                  15

Ser Leu Arg Leu Ser Cys Ala Ala Ser
            20                  25

<210>  3
<211>  25
<212>  PRT
<213>  Mus musculus

<400>  3

Gln Val Gln Leu Val Glu Ser Gly Gly Gly Leu Val Gln Pro Gly Gly
1               5                   10                  15

Ser Leu Arg Leu Ser Cys Ala Ala Ser
            20                  25

<210>  4
<211>  25
<212>  PRT
<213>  Mus musculus

<400>  4

Gln Val Gln Leu Val Glu Ser Gly Gly Gly Leu Val Gln Ala Gly Gly
1               5                   10                  15

Ser Leu Arg Leu Ser Cys Ala Ala Ser
            20                  25

<210>  5
<211>  25
<212>  PRT
<213>  Mus musculus

<400>  5

Gln Leu Gln Leu Val Glu Ser Gly Gly Gly Leu Val Gln Pro Gly Gly
1               5                   10                  15

Ser Leu Arg Leu Ser Cys Ala Ala Ser
            20                  25

<210>  6
<211>  8
<212>  PRT
<213>  Mus musculus

<400>  6

Gly Ser Ile Phe Ser Ala Asn Leu
1               5

<210>  7
<211>  8
<212>  PRT
<213>  Mus musculus

<400>  7

Gly Gly Thr Phe Ile Thr Tyr Ala
1               5

<210>  8
<211>  8
<212>  PRT
<213>  Mus musculus

<400>  8

Gly Arg Ala Phe Leu Thr Tyr Ala
1               5

<210>  9
<211>  8
<212>  PRT
<213>  Mus musculus

<400>  9

Gly Arg Pro Phe Ile Thr Tyr Ala
1               5

<210>  10
<211>  8
<212>  PRT
<213>  Mus musculus

<400>  10

Gly Arg Thr Phe Ser Thr His Ser
1               5

<210>  11
<211>  17
<212>  PRT
<213>  Mus musculus

<400>  11

Ile Asp Trp Tyr Arg Gln Pro Pro Gly Lys Gln Arg Glu Leu Val Ala
1               5                   10                  15

Val

<210>  12
<211>  17
<212>  PRT
<213>  Mus musculus

<400>  12

Met Gly Trp Phe Arg Gln Ala Pro Gly Lys Asp Arg Glu Phe Val Ala
1               5                   10                  15

Gly

<210>  13
<211>  17
<212>  PRT
<213>  Mus musculus

<400>  13

Met Gly Trp Phe Arg Gln Ala Pro Gly Lys Glu Arg Glu Phe Val Ala
1               5                   10                  15

Ala

<210>  14
<211>  17

<212>  PRT
<213>  Mus musculus

<400>  14

Met His Trp Phe Arg Gln Ala Pro Glu Lys Glu Arg Val Phe Val Ala
1               5                   10                  15

Ala

<210>  15
<211>  7
<212>  PRT
<213>  Mus musculus

<400>  15

Ile Ser Ser Arg Gly Ile Thr
1               5

<210>  16
<211>  8
<212>  PRT
<213>  Mus musculus

<400>  16

Ile Ser Trp Ser Gly Ser Ser Thr
1               5

<210>  17
<211>  8
<212>  PRT
<213>  Mus musculus

<400>  17

Ile Asn Trp Ser Gly Ser Ile Thr
1               5

<210>  18
<211>  8
<212>  PRT
<213>  Mus musculus

<400>  18

Ile Asn Trp Asn Gly Asp Ser Thr
1               5

<210>  19
<211>  38
<212>  PRT
<213>  Mus musculus

<400>  19

Ser Tyr Ala Asp Ser Val Lys Gly Arg Phe Thr Ile Ser Arg Asp Asn
1               5                   10                  15

Ala Lys Asn Thr Val Tyr Leu Gln Met Asn Ser Leu Lys Pro Glu Asp
            20                  25                  30

Thr Ala Val Tyr Tyr Cys
        35

<210>  20
<211>  38
<212>  PRT
<213>  Mus musculus

<400>  20

Tyr Tyr Thr Asp Ser Val Lys Gly Arg Phe Thr Ile Ser Arg Asp Asn
1               5                   10                  15

Ala Lys Asn Thr Val Tyr Leu Gln Met Asn Ser Leu Lys Pro Gly Asp
            20                  25                  30

Thr Ala Val Tyr Tyr Cys
        35

<210>  21
<211>  38
<212>  PRT
<213>  Mus musculus

<400>  21

Tyr Tyr Ala Asp Ser Val Lys Gly Arg Phe Thr Ile Ser Arg Asp Asn
1               5                   10                  15

Ala Lys Asn Met Val Tyr Leu Gln Met Asn Ser Leu Lys Pro Glu Asp
            20                  25                  30

Thr Ala Val Tyr Tyr Cys
        35

<210>  22
<211>  38
<212>  PRT
<213>  Mus musculus

<400>  22

Ala Tyr Ala Asp Ser Val Lys Gly Arg Phe Thr Ile Ser Arg Asp Asn
1               5                   10                  15

Ala Lys Asn Ala Ala Tyr Leu Gln Met Asn Ser Leu Lys Pro Asp Asp

20                  25                  30

Thr Ala Val Tyr Tyr Cys
        35

<210>  23
<211>  38
<212>  PRT
<213>  Mus musculus

<400>  23

Tyr Tyr Thr Asp Ser Val Lys Gly Arg Phe Thr Ile Ser Arg Asp Asn
1               5                   10                  15

Ala Gln Asn Thr Val Tyr Leu Gln Met Asn Ser Arg Lys Pro Gly Asp
            20                  25                  30

Thr Ala Val Tyr Tyr Cys
        35

<210>  24
<211>  38
<212>  PRT
<213>  Mus musculus

<400>  24

Tyr Tyr Ala Asp Ser Val Lys Gly Arg Phe Thr Ile Ser Arg Asp Asn
1               5                   10                  15

Ser Lys Asn Thr Val Asp Leu Gln Met Asn Asn Leu Lys Pro Glu Asp
            20                  25                  30

Thr Ala Val Tyr Val Cys
        35

<210>  25
<211>  10
<212>  PRT
<213>  Mus musculus

<400>  25

His Leu Phe His Thr Asp Gly Leu Gly Tyr
1               5                   10

<210>  26
<211>  18
<212>  PRT
<213>  Mus musculus

<400>  26

Ala Ala Lys Ile Ser Gly Ala Thr Arg Glu His Leu Met Thr Ser Tyr
1               5                   10                  15

Asp Tyr

<210>  27
<211>  18
<212>  PRT
<213>  Mus musculus

<400>  27

Ala Ala Lys Val Ser Gly Ala Thr Arg Asp His Leu Met Thr Thr Tyr
1               5                   10                  15

Asp Tyr

<210>  28
<211>  17
<212>  PRT
<213>  Mus musculus

<400>  28

Ala Ser Lys Arg Thr Ala Val Ala Pro Arg Ala Leu Asn Asp Tyr Asp
1               5                   10                  15

Phe

<210>  29
<211>  18
<212>  PRT
<213>  Mus musculus

<400>  29

Ala Ala Lys Ile Ser Gly Ala Thr Arg Glu His Leu Arg Thr Ser Tyr
1               5                   10                  15

Asp Tyr

<210>  30
<211>  16
<212>  PRT
<213>  Mus musculus

<400>  30

Ala Ala Arg Pro Gly Ala Pro Ser Thr Ile Val Ala Met Leu Asp Tyr
1               5                   10                  15

<210>  31
<211>  11
<212>  PRT
<213>  Mus musculus

<400>  31

Trp Gly Gln Gly Thr Gln Val Thr Val Ser Ser
1               5                   10


<210>  32
<211>  11
<212>  PRT
<213>  Mus musculus

<400>  32

Gly Gly Gln Gly Thr Gln Val Thr Val Ser Ser
1               5                   10


<210>  33
<211>  116
<212>  PRT
<213>  Mus musculus

<400>  33

Gln Leu Gln Leu Val Glu Ser Gly Gly Asp Leu Val Gln Pro Gly Gly
1               5                   10                  15

Ser Leu Arg Leu Ser Cys Ala Ala Ser Gly Ser Ile Phe Ser Ala Asn
            20                  25                  30

Leu Ile Asp Trp Tyr Arg Gln Pro Pro Gly Lys Gln Arg Glu Leu Val
        35                  40                  45

Ala Val Ile Ser Ser Arg Gly Ile Thr Ser Tyr Ala Asp Ser Val Lys
    50                  55                  60

Gly Arg Phe Thr Ile Ser Arg Asp Asn Ala Lys Asn Thr Val Tyr Leu
65                  70                  75                  80

Gln Met Asn Ser Leu Lys Pro Glu Asp Thr Ala Val Tyr Tyr Cys His
                85                  90                  95

Leu Phe His Thr Asp Gly Leu Gly Tyr Trp Gly Gln Gly Thr Gln Val
            100                 105                 110

Thr Val Ser Ser
        115


<210>  34

<211>  125
<212>  PRT
<213>  Mus musculus

<400>  34

Gln Leu Gln Leu Val Glu Ser Gly Gly Gly Leu Val Gln Ala Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Asp Arg Glu Phe Val
        35                  40                  45

Ala Gly Ile Ser Trp Ser Gly Ser Ser Thr Tyr Tyr Thr Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Gly Asp Thr Ala Val Tyr Tyr Cys
                85                  90                  95

Ala Ala Lys Ile Ser Gly Ala Thr Arg Glu His Leu Met Thr Ser Tyr
            100                 105                 110

Asp Tyr Trp Gly Gln Gly Thr Gln Val Thr Val Ser Ser
        115                 120                 125


<210>  35
<211>  125
<212>  PRT
<213>  Mus musculus

<400>  35

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Ala Phe Leu Thr Tyr
            20                  25                  30

Ala Met Gly Trp Phe Arg Gln Ala Pro Gly Lys Glu Arg Glu Phe Val
        35                  40                  45

Ala Ala Ile Ser Trp Ser Gly Ser Ser Thr Tyr Tyr Ala Asp Ser Val
    50                  55                  60

Lys Gly Arg Phe Thr Ile Ser Arg Asp Asn Ala Lys Asn Met Val Tyr
65                  70                  75                  80

Leu Gln Met Asn Ser Leu Lys Pro Glu Asp Thr Ala Val Tyr Tyr Cys
                85                  90                  95

Ala Ala Lys Val Ser Gly Ala Thr Arg Asp His Leu Met Thr Thr Tyr
            100                 105                 110

Asp Tyr Trp Gly Gln Gly Thr Gln Val Thr Val Ser Ser
        115                 120                 125

<210>  36
<211>  124
<212>  PRT
<213>  Mus musculus

<400>  36

Gln Val Gln Leu Val Glu Ser Gly Gly Gly Leu Val Gln Ala Gly Gly
1               5                   10                  15

Ser Leu Arg Leu Ser Cys Ala Ala Ser Gly Arg Pro Phe Ile Thr Tyr
            20                  25                  30

Ala Met Gly Trp Phe Arg Gln Ala Pro Gly Lys Glu Arg Glu Phe Val
        35                  40                  45

Ala Ala Ile Asn Trp Ser Gly Ser Ile Thr Ala Tyr Ala Asp Ser Val
    50                  55                  60

Lys Gly Arg Phe Thr Ile Ser Arg Asp Asn Ala Lys Asn Ala Ala Tyr
65                  70                  75                  80

Leu Gln Met Asn Ser Leu Lys Pro Asp Asp Thr Ala Val Tyr Tyr Cys
                85                  90                  95

Ala Ser Lys Arg Thr Ala Val Ala Pro Arg Ala Leu Asn Asp Tyr Asp
            100                 105                 110

Phe Trp Gly Gln Gly Thr Gln Val Thr Val Ser Ser
        115                 120

<210>  37
<211>  125
<212>  PRT
<213>  Mus musculus

<400>  37

Gln Leu Gln Leu Val Glu Ser Gly Gly Gly Leu Val Gln Pro Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Asp Arg Glu Phe Val
        35                  40                  45

Ala Gly Ile Ser Trp Ser Gly Ser Ser Thr Tyr Tyr Thr Asp Ser Val
    50                  55                  60

Lys Gly Arg Phe Thr Ile Ser Arg Asp Asn Ala Gln Asn Thr Val Tyr
65                  70                  75                  80

Leu Gln Met Asn Ser Arg Lys Pro Gly Asp Thr Ala Val Tyr Tyr Cys
                85                  90                  95

Ala Ala Lys Ile Ser Gly Ala Thr Arg Glu His Leu Arg Thr Ser Tyr
            100                 105                 110

Asp Tyr Gly Gly Gln Gly Thr Gln Val Thr Val Ser Ser
        115                 120                 125

<210> 38
<211> 123
<212> PRT
<213> Mus musculus

<400> 38

Gln Leu Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Thr His
            20                  25                  30

Ser Met His Trp Phe Arg Gln Ala Pro Glu Lys Glu Arg Val Phe Val
        35                  40                  45

Ala Ala Ile Asn Trp Asn Gly Asp Ser Thr Tyr Tyr Ala Asp Ser Val
    50                  55                  60

Lys Gly Arg Phe Thr Ile Ser Arg Asp Asn Ser Lys Asn Thr Val Asp
65                  70                  75                  80

Leu Gln Met Asn Asn Leu Lys Pro Glu Asp Thr Ala Val Tyr Val Cys
                85                  90                  95

Ala Ala Arg Pro Gly Ala Pro Ser Thr Ile Val Ala Met Leu Asp Tyr
            100                 105                 110

Trp Gly Gln Gly Thr Gln Val Thr Val Ser Ser
        115                 120

<210>  39
<211>  116
<212>  PRT
<213>  Artificial Sequence

<220>
<223>  hu1A7V3

<220>
<221>  CHAIN
<222>  (1)..(116)

<400>  39

Gln Val Gln Leu Val Glu Ser Gly Gly Gly Leu Val Gln Pro Gly Gly
1               5                   10                  15

Ser Leu Arg Leu Ser Cys Ala Ala Ser Gly Ser Ile Phe Ser Ala Asn
            20                  25                  30

Leu Ile Asp Trp Tyr Arg Gln Ala Pro Gly Lys Gly Leu Glu Leu Val
        35                  40                  45

Ala Val Ile Ser Ser Arg Gly Ile Thr Ser Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys His
                85                  90                  95

Leu Phe His Thr Asp Gly Leu Gly Tyr Trp Gly Gln Gly Thr Leu Val
            100                 105                 110

Thr Val Ser Ser
        115

<210>  40
<211>  125
<212>  PRT
<213>  Artificial Sequence

<220>
<223>  hu1D1V3

<220>
<221>  CHAIN

<222>  (1)..(125)

<400>  40

Gln Val Gln Leu Val Glu Ser Gly Gly Gly Leu Val Gln Pro Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Gly Leu Glu Phe Val
        35                  40                  45

Ala Gly Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Ile Ser Gly Ala Thr Arg Glu His Leu Met Thr Ser Tyr
            100                 105                 110

Asp Tyr Trp Gly Gln Gly Thr Leu Val Thr Val Ser Ser
        115                 120                 125

<210>  41
<211>  125
<212>  PRT
<213>  Artificial Sequence

<220>
<223>  hu1D7V3

<220>
<221>  CHAIN
<222>  (1)..(125)

<400>  41

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Ala Phe Leu Thr Tyr
            20                  25                  30

Ala Met Gly Trp Phe Arg Gln Ala Pro Gly Lys Gly Leu Glu Phe Val
        35                  40                  45

Ala Ala Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Val Ser Gly Ala Thr Arg Asp His Leu Met Thr Thr Tyr
            100                 105                 110

Asp Tyr Trp Gly Gln Gly Thr Leu Val Thr Val Ser Ser
        115                 120                 125

<210>  42
<211>  124
<212>  PRT
<213>  Artificial Sequence

<220>
<223>  hu1G10V3

<220>
<221>  CHAIN
<222>  (1)..(124)

<400>  42

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Pro Phe Ile Thr Tyr
            20                  25                  30

Ala Met Gly Trp Phe Arg Gln Ala Pro Gly Lys Gly Leu Glu Phe Val
        35                  40                  45

Ala Ala Ile Asn Trp Ser Gly Ser Ile Thr Ala Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Lys Arg Thr Ala Val Ala Pro Arg Ala Leu Asn Asp Tyr Asp
            100                 105                 110

Phe Trp Gly Gln Gly Thr Leu Val Thr Val Ser Ser
        115                 120

<210>  43
<211>  125
<212>  PRT
<213>  Artificial Sequence

<220>
<223>  hu5A8V3

<220>
<221>  CHAIN
<222>  (1)..(125)

<400>  43

Gln Val Gln Leu Val Glu Ser Gly Gly Gly Leu Val Gln Pro Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Gly Leu Glu Phe Val
        35                  40                  45

Ala Gly Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Ile Ser Gly Ala Thr Arg Glu His Leu Arg Thr Ser Tyr
            100                 105                 110

Asp Tyr Trp Gly Gln Gly Thr Leu Val Thr Val Ser Ser
        115                 120                 125

<210>  44
<211>  123
<212>  PRT
<213>  Artificial Sequence

<220>
<223>  hu6F1V3

<220>
<221>  CHAIN
<222>  (1)..(123)

<400>  44

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Thr His
            20                  25                  30

Ser Met Gly Trp Phe Arg Gln Ala Pro Gly Lys Gly Leu Glu Phe Val
        35                  40                  45

Ala Ala Ile Asn Trp Asn Gly Asp Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Arg Pro Gly Ala Pro Ser Thr Ile Val Ala Met Leu Asp Tyr
            100                 105                 110

Trp Gly Gln Gly Thr Leu Val Thr Val Ser Ser
        115                 120


<210>  45
<211>  231
<212>  PRT
<213>  Artificial Sequence

<220>
<223>  Natural IgG1 FC, K removed


<220>
<221>  PEPTIDE
<222>  (1)..(231)

<400>  45

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val

50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly
225                 230

<210>  46
<211>  228
<212>  PRT
<213>  Homo sapiens

<400>  46

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val

35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly
225

<210>  47
<211>  231
<212>  PRT
<213>  Artificial Sequence

<220>
<223>  Fc region of immunoglobulin (attenuated, K removed)

<220>
<221>  PEPTIDE
<222>  (1)..(231)

<400>  47

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Ala Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Gly Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly
225                 230

<210>  48
<211>  231
<212>  PRT
<213>  Artificial Sequence

<220>
<223>  Fc region of immunoglobulin (attenuated, K removed)


<220>
<221>  PEPTIDE
<222>  (1)..(231)

<400>  48

Glu Pro Lys Ser Ser Asp Lys Thr His Thr Cys Pro Pro Cys Pro Ala
1               5                   10                  15

Pro Glu Leu Leu Gly Gly Pro Asp Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Glu Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly
225                 230

<210>  49
<211>  815
<212>  PRT
<213>  Artificial Sequence

<220>
<223>  PDL1-HSA

<220>
<221>  PEPTIDE
<222>  (1)..(815)

<400>  49

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg Thr Gly Gly Gly
    210                 215                 220

Gly Ser Gly Gly Gly Gly Asp Ala His Lys Ser Glu Val Ala His Arg
225                 230                 235                 240

Phe Lys Asp Leu Gly Glu Glu Asn Phe Lys Ala Leu Val Leu Ile Ala
                245                 250                 255

Phe Ala Gln Tyr Leu Gln Gln Cys Pro Phe Glu Asp His Val Lys Leu
            260                 265                 270

Val Asn Glu Val Thr Glu Phe Ala Lys Thr Cys Val Ala Asp Glu Ser
        275                 280                 285

Ala Glu Asn Cys Asp Lys Ser Leu His Thr Leu Phe Gly Asp Lys Leu
    290                 295                 300

Cys Thr Val Ala Thr Leu Arg Glu Thr Tyr Gly Glu Met Ala Asp Cys
305                 310                 315                 320

Cys Ala Lys Gln Glu Pro Glu Arg Asn Glu Cys Phe Leu Gln His Lys
                325                 330                 335

Asp Asp Asn Pro Asn Leu Pro Arg Leu Val Arg Pro Glu Val Asp Val
            340                 345                 350

Met Cys Thr Ala Phe His Asp Asn Glu Glu Thr Phe Leu Lys Lys Tyr
        355                 360                 365

Leu Tyr Glu Ile Ala Arg Arg His Pro Tyr Phe Tyr Ala Pro Glu Leu
    370                 375                 380

Leu Phe Phe Ala Lys Arg Tyr Lys Ala Ala Phe Thr Glu Cys Cys Gln
385                 390                 395                 400

Ala Ala Asp Lys Ala Ala Cys Leu Leu Pro Lys Leu Asp Glu Leu Arg
                405                 410                 415

Asp Glu Gly Lys Ala Ser Ser Ala Lys Gln Arg Leu Lys Cys Ala Ser
            420                 425                 430

Leu Gln Lys Phe Gly Glu Arg Ala Phe Lys Ala Trp Ala Val Ala Arg
        435                 440                 445

Leu Ser Gln Arg Phe Pro Lys Ala Glu Phe Ala Glu Val Ser Lys Leu
    450                 455                 460

Val Thr Asp Leu Thr Lys Val His Thr Glu Cys Cys His Gly Asp Leu
465                 470                 475                 480

Leu Glu Cys Ala Asp Asp Arg Ala Asp Leu Ala Lys Tyr Ile Cys Glu
                485                 490                 495

Asn Gln Asp Ser Ile Ser Ser Lys Leu Lys Glu Cys Cys Glu Lys Pro
            500                 505                 510

Leu Leu Glu Lys Ser His Cys Ile Ala Glu Val Glu Asn Asp Glu Met
        515                 520                 525

Pro Ala Asp Leu Pro Ser Leu Ala Ala Asp Phe Val Glu Ser Lys Asp
    530                 535                 540

Val Cys Lys Asn Tyr Ala Glu Ala Lys Asp Val Phe Leu Gly Met Phe
545                 550                 555                 560

Leu Tyr Glu Tyr Ala Arg Arg His Pro Asp Tyr Ser Val Val Leu Leu
                565                 570                 575

Leu Arg Leu Ala Lys Thr Tyr Glu Thr Thr Leu Glu Lys Cys Cys Ala
            580                 585                 590

Ala Ala Asp Pro His Glu Cys Tyr Ala Lys Val Phe Asp Glu Phe Lys
        595                 600                 605

Pro Leu Val Glu Glu Pro Gln Asn Leu Ile Lys Gln Asn Cys Glu Leu
    610                 615                 620

Phe Glu Gln Leu Gly Glu Tyr Lys Phe Gln Asn Ala Leu Leu Val Arg
625                 630                 635                 640

Tyr Thr Lys Lys Val Pro Gln Val Ser Thr Pro Thr Leu Val Glu Val
                645                 650                 655

Ser Arg Asn Leu Gly Lys Val Gly Ser Lys Cys Cys Lys His Pro Glu
            660                 665                 670

Ala Lys Arg Met Pro Cys Ala Glu Asp Tyr Leu Ser Val Val Leu Asn
        675                 680                 685

Gln Leu Cys Val Leu His Glu Lys Thr Pro Val Ser Asp Arg Val Thr
    690                 695                 700

Lys Cys Cys Thr Glu Ser Leu Val Asn Arg Arg Pro Cys Phe Ser Ala
705                 710                 715                 720

Leu Glu Val Asp Glu Thr Tyr Val Pro Lys Glu Phe Asn Ala Glu Thr
                725                 730                 735

Phe Thr Phe His Ala Asp Ile Cys Thr Leu Ser Glu Lys Glu Arg Gln
            740                 745                 750

Ile Lys Lys Gln Thr Ala Leu Val Glu Leu Val Lys His Lys Pro Lys
        755                 760                 765

Ala Thr Lys Glu Gln Leu Lys Ala Val Met Asp Asp Phe Ala Ala Phe
    770                 775                 780

Val Glu Lys Cys Cys Lys Ala Asp Asp Lys Glu Thr Cys Phe Ala Glu
785                 790                 795                 800

Glu Gly Lys Lys Leu Val Ala Ala Ser Gln Ala Ala Leu Gly Leu
                805                 810                 815

<210>  50
<211>  459
<212>  PRT
<213>  Artificial Sequence

<220>
<223>  PDL1-FC

<220>
<221>  PEPTIDE
<222>  (1)..(459)

<400>  50

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu Pro Leu Ala His Pro Pro Asn Glu Arg Thr Asp Ile Glu
    210                 215                 220

Gly Arg Met Asp Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn

275                 280                 285

Trp Tyr Val Asp Gly Val Glu Val His Asn Ala Lys Thr Lys Pro Arg
    290                 295                 300

Glu Glu Gln Tyr Ala Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Ser Arg Asp
        355                 360                 365

Glu Leu Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  51
<211>  381
<212>  PRT
<213>  Artificial Sequence

<220>
<223>  PD1-FC


<220>
<221>  PEPTIDE
<222>  (1)..(381)

<400>  51

Leu Asp Ser Pro Asp Arg Pro Trp Asn Pro Pro Thr Phe Ser Pro Ala

1               5                   10                  15

Leu Leu Val Val Thr Glu Gly Asp Asn Ala Thr Phe Thr Cys Ser Phe
            20                  25                  30

Ser Asn Thr Ser Glu Ser Phe Val Leu Asn Trp Tyr Arg Met Ser Pro
        35                  40                  45

Ser Asn Gln Thr Asp Lys Leu Ala Ala Phe Pro Glu Asp Arg Ser Gln
    50                  55                  60

Pro Gly Gln Asp Cys Arg Phe Arg Val Thr Gln Leu Pro Asn Gly Arg
65                  70                  75                  80

Asp Phe His Met Ser Val Val Arg Ala Arg Arg Asn Asp Ser Gly Thr
                85                  90                  95

Tyr Leu Cys Gly Ala Ile Ser Leu Ala Pro Lys Ala Gln Ile Lys Glu
            100                 105                 110

Ser Leu Arg Ala Glu Leu Arg Val Thr Glu Arg Arg Ala Glu Val Pro
        115                 120                 125

Thr Ala His Pro Ser Pro Ser Pro Arg Pro Ala Gly Gln Phe Gln Asp
    130                 135                 140

Ile Glu Gly Arg Met Asp Pro Lys Ser Cys Asp Lys Thr His Thr Cys
145                 150                 155                 160

Pro Pro Cys Pro Ala Pro Glu Leu Leu Gly Gly Pro Ser Val Phe Leu
                165                 170                 175

Phe Pro Pro Lys Pro Lys Asp Thr Leu Met Ile Ser Arg Thr Pro Glu
            180                 185                 190

Val Thr Cys Val Val Val Asp Val Ser His Glu Asp Pro Glu Val Lys
        195                 200                 205

Phe Asn Trp Tyr Val Asp Gly Val Glu Val His Asn Ala Lys Thr Lys
    210                 215                 220

Pro Arg Glu Glu Gln Tyr Ala Ser Thr Tyr Arg Val Val Ser Val Leu
225                 230                 235                 240

Thr Val Leu His Gln Asp Trp Leu Asn Gly Lys Glu Tyr Lys Cys Lys
                245                 250                 255

Val Ser Asn Lys Ala Leu Pro Ala Pro Ile Glu Lys Thr Ile Ser Lys
            260                 265                 270

Ala Lys Gly Gln Pro Arg Glu Pro Gln Val Tyr Thr Leu Pro Pro Ser
        275                 280                 285

Arg Asp Glu Leu Thr Lys Asn Gln Val Ser Leu Thr Cys Leu Val Lys
    290                 295                 300

Gly Phe Tyr Pro Ser Asp Ile Ala Val Glu Trp Glu Ser Asn Gly Gln
305                 310                 315                 320

Pro Glu Asn Asn Tyr Lys Thr Thr Pro Pro Val Leu Asp Ser Asp Gly
                325                 330                 335

Ser Phe Phe Leu Tyr Ser Lys Leu Thr Val Asp Lys Ser Arg Trp Gln
            340                 345                 350

Gln Gly Asn Val Phe Ser Cys Ser Val Met His Glu Ala Leu His Asn
        355                 360                 365

His Tyr Thr Gln Lys Ser Leu Ser Leu Ser Pro Gly Lys
    370                 375                 380

<210> 52
<211> 815
<212> PRT
<213> Artificial Sequence

<220>
<223> mPDL1-HSA

<220>
<221> PEPTIDE
<222> (1)..(815)

<400> 52

Phe Thr Ile Thr Ala Pro Lys Asp Leu Tyr Val Val Glu Tyr Gly Ser
1               5                   10                  15

Asn Val Thr Met Glu Cys Arg Phe Pro Val Glu Arg Glu Leu Asp Leu
            20                  25                  30

Leu Ala Leu Val Val Tyr Trp Glu Lys Glu Asp Glu Gln Val Ile Gln
        35                  40                  45

Phe Val Ala Gly Glu Glu Asp Leu Lys Pro Gln His Ser Asn Phe Arg
    50                  55                  60

Gly Arg Ala Ser Leu Pro Lys Asp Gln Leu Leu Lys Gly Asn Ala Ala
65                  70                  75                  80

Leu Gln Ile Thr Asp Val Lys Leu Gln Asp Ala Gly Val Tyr Cys Cys
                85                  90                  95

Ile Ile Ser Tyr Gly Gly Ala Asp Tyr Lys Arg Ile Thr Leu Lys Val
            100                 105                 110

Asn Ala Pro Tyr Arg Lys Ile Asn Gln Arg Ile Ser Val Asp Pro Ala
        115                 120                 125

Thr Ser Glu His Glu Leu Ile Cys Gln Ala Glu Gly Tyr Pro Glu Ala
    130                 135                 140

Glu Val Ile Trp Thr Asn Ser Asp His Gln Pro Val Ser Gly Lys Arg
145                 150                 155                 160

Ser Val Thr Thr Ser Arg Thr Glu Gly Met Leu Leu Asn Val Thr Ser
                165                 170                 175

Ser Leu Arg Val Asn Ala Thr Ala Asn Asp Val Phe Tyr Cys Thr Phe
            180                 185                 190

Trp Arg Ser Gln Pro Gly Gln Asn His Thr Ala Glu Leu Ile Ile Pro
        195                 200                 205

Glu Leu Pro Ala Thr His Pro Pro Gln Asn Arg Thr His Gly Gly Gly
    210                 215                 220

Gly Ser Gly Gly Gly Gly Asp Ala His Lys Ser Glu Val Ala His Arg
225                 230                 235                 240

Phe Lys Asp Leu Gly Glu Glu Asn Phe Lys Ala Leu Val Leu Ile Ala
                245                 250                 255

Phe Ala Gln Tyr Leu Gln Gln Cys Pro Phe Glu Asp His Val Lys Leu
            260                 265                 270

Val Asn Glu Val Thr Glu Phe Ala Lys Thr Cys Val Ala Asp Glu Ser
        275                 280                 285

Ala Glu Asn Cys Asp Lys Ser Leu His Thr Leu Phe Gly Asp Lys Leu
    290                 295                 300

Cys Thr Val Ala Thr Leu Arg Glu Thr Tyr Gly Glu Met Ala Asp Cys
305                 310                 315                 320

Cys Ala Lys Gln Glu Pro Glu Arg Asn Glu Cys Phe Leu Gln His Lys
                325                 330                 335

Asp Asp Asn Pro Asn Leu Pro Arg Leu Val Arg Pro Glu Val Asp Val
            340                 345                 350

Met Cys Thr Ala Phe His Asp Asn Glu Glu Thr Phe Leu Lys Lys Tyr
        355                 360                 365

Leu Tyr Glu Ile Ala Arg Arg His Pro Tyr Phe Tyr Ala Pro Glu Leu
    370                 375                 380

Leu Phe Phe Ala Lys Arg Tyr Lys Ala Ala Phe Thr Glu Cys Cys Gln
385                 390                 395                 400

Ala Ala Asp Lys Ala Ala Cys Leu Leu Pro Lys Leu Asp Glu Leu Arg
                405                 410                 415

Asp Glu Gly Lys Ala Ser Ser Ala Lys Gln Arg Leu Lys Cys Ala Ser
            420                 425                 430

Leu Gln Lys Phe Gly Glu Arg Ala Phe Lys Ala Trp Ala Val Ala Arg
        435                 440                 445

Leu Ser Gln Arg Phe Pro Lys Ala Glu Phe Ala Glu Val Ser Lys Leu
    450                 455                 460

Val Thr Asp Leu Thr Lys Val His Thr Glu Cys Cys His Gly Asp Leu
465                 470                 475                 480

Leu Glu Cys Ala Asp Asp Arg Ala Asp Leu Ala Lys Tyr Ile Cys Glu
                485                 490                 495

Asn Gln Asp Ser Ile Ser Ser Lys Leu Lys Glu Cys Cys Glu Lys Pro
            500                 505                 510

Leu Leu Glu Lys Ser His Cys Ile Ala Glu Val Glu Asn Asp Glu Met
        515                 520                 525

Pro Ala Asp Leu Pro Ser Leu Ala Ala Asp Phe Val Glu Ser Lys Asp
    530                 535                 540

Val Cys Lys Asn Tyr Ala Glu Ala Lys Asp Val Phe Leu Gly Met Phe
545                 550                 555                 560

Leu Tyr Glu Tyr Ala Arg Arg His Pro Asp Tyr Ser Val Val Leu Leu
                565                 570                 575

Leu Arg Leu Ala Lys Thr Tyr Glu Thr Thr Leu Glu Lys Cys Cys Ala
            580                 585                 590

Ala Ala Asp Pro His Glu Cys Tyr Ala Lys Val Phe Asp Glu Phe Lys
        595                 600                 605

Pro Leu Val Glu Glu Pro Gln Asn Leu Ile Lys Gln Asn Cys Glu Leu
    610                 615                 620

Phe Glu Gln Leu Gly Glu Tyr Lys Phe Gln Asn Ala Leu Leu Val Arg
625                 630                 635                 640

Tyr Thr Lys Lys Val Pro Gln Val Ser Thr Pro Thr Leu Val Glu Val
                645                 650                 655

Ser Arg Asn Leu Gly Lys Val Gly Ser Lys Cys Cys Lys His Pro Glu
            660                 665                 670

Ala Lys Arg Met Pro Cys Ala Glu Asp Tyr Leu Ser Val Val Leu Asn
        675                 680                 685

Gln Leu Cys Val Leu His Glu Lys Thr Pro Val Ser Asp Arg Val Thr
    690                 695                 700

Lys Cys Cys Thr Glu Ser Leu Val Asn Arg Arg Pro Cys Phe Ser Ala
705                 710                 715                 720

Leu Glu Val Asp Glu Thr Tyr Val Pro Lys Glu Phe Asn Ala Glu Thr
                725                 730                 735

Phe Thr Phe His Ala Asp Ile Cys Thr Leu Ser Glu Lys Glu Arg Gln
            740                 745                 750

Ile Lys Lys Gln Thr Ala Leu Val Glu Leu Val Lys His Lys Pro Lys
        755                 760                 765

Ala Thr Lys Glu Gln Leu Lys Ala Val Met Asp Asp Phe Ala Ala Phe
    770                 775                 780

Val Glu Lys Cys Cys Lys Ala Asp Asp Lys Glu Thr Cys Phe Ala Glu
785                 790                 795                 800

Glu Gly Lys Lys Leu Val Ala Ala Ser Gln Ala Ala Leu Gly Leu
                805                 810                 815

<210>  53

<211>  347
<212>  PRT
<213>  Artificial Sequence

<220>
<223>  Anti-PDL1-1A7V3-Fc


<220>
<221>  CHAIN
<222>  (1)..(347)

<400>  53

Gln Val Gln Leu Val Glu Ser Gly Gly Gly Leu Val Gln Pro Gly Gly
1               5                   10                  15

Ser Leu Arg Leu Ser Cys Ala Ala Ser Gly Ser Ile Phe Ser Ala Asn
            20                  25                  30

Leu Ile Asp Trp Tyr Arg Gln Ala Pro Gly Lys Gly Leu Glu Leu Val
        35                  40                  45

Ala Val Ile Ser Ser Arg Gly Ile Thr Ser Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys His
                85                  90                  95

Leu Phe His Thr Asp Gly Leu Gly Tyr Trp Gly Gln Gly Thr Leu Val
            100                 105                 110

Thr Val Ser Ser Glu Pro Lys Ser Cys Asp Lys Thr His Thr Cys Pro
        115                 120                 125

Pro Cys Pro Ala Pro Glu Leu Leu Gly Gly Pro Ser Val Phe Leu Phe
    130                 135                 140

Pro Pro Lys Pro Lys Asp Thr Leu Met Ile Ser Arg Thr Pro Glu Val
145                 150                 155                 160

Thr Cys Val Val Val Asp Val Ser His Glu Asp Pro Glu Val Lys Phe
                165                 170                 175

Asn Trp Tyr Val Asp Gly Val Glu Val His Asn Ala Lys Thr Lys Pro
            180                 185                 190

Arg Glu Glu Gln Tyr Asn Ser Thr Tyr Arg Val Val Ser Val Leu Thr

195                 200                 205

Val Leu His Gln Asp Trp Leu Asn Gly Lys Glu Tyr Lys Cys Lys Val
    210                 215                 220

Ser Asn Lys Ala Leu Pro Ala Pro Ile Glu Lys Thr Ile Ser Lys Ala
225                 230                 235                 240

Lys Gly Gln Pro Arg Glu Pro Gln Val Tyr Thr Leu Pro Pro Ser Arg
                245                 250                 255

Asp Glu Leu Thr Lys Asn Gln Val Ser Leu Thr Cys Leu Val Lys Gly
            260                 265                 270

Phe Tyr Pro Ser Asp Ile Ala Val Glu Trp Glu Ser Asn Gly Gln Pro
        275                 280                 285

Glu Asn Asn Tyr Lys Thr Thr Pro Pro Val Leu Asp Ser Asp Gly Ser
    290                 295                 300

Phe Phe Leu Tyr Ser Lys Leu Thr Val Asp Lys Ser Arg Trp Gln Gln
305                 310                 315                 320

Gly Asn Val Phe Ser Cys Ser Val Met His Glu Ala Leu His Asn His
                325                 330                 335

Tyr Thr Gln Lys Ser Leu Ser Leu Ser Pro Gly
            340                 345

<210>  54
<211>  356
<212>  PRT
<213>  Artificial Sequence

<220>
<223>  Anti-PDL1-1D1V3-Fc


<220>
<221>  CHAIN
<222>  (1)..(356)

<400>  54

Gln Val Gln Leu Val Glu Ser Gly Gly Gly Leu Val Gln Pro Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Gly Leu Glu Phe Val

35                  40                  45

Ala Gly Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Ile Ser Gly Ala Thr Arg Glu His Leu Met Thr Ser Tyr
            100                 105                 110

Asp Tyr Trp Gly Gln Gly Thr Leu Val Thr Val Ser Ser Glu Pro Lys
        115                 120                 125

Ser Cys Asp Lys Thr His Thr Cys Pro Pro Cys Pro Ala Pro Glu Leu
    130                 135                 140

Leu Gly Gly Pro Ser Val Phe Leu Phe Pro Pro Lys Pro Lys Asp Thr
145                 150                 155                 160

Leu Met Ile Ser Arg Thr Pro Glu Val Thr Cys Val Val Val Asp Val
                165                 170                 175

Ser His Glu Asp Pro Glu Val Lys Phe Asn Trp Tyr Val Asp Gly Val
            180                 185                 190

Glu Val His Asn Ala Lys Thr Lys Pro Arg Glu Glu Gln Tyr Asn Ser
        195                 200                 205

Thr Tyr Arg Val Val Ser Val Leu Thr Val Leu His Gln Asp Trp Leu
    210                 215                 220

Asn Gly Lys Glu Tyr Lys Cys Lys Val Ser Asn Lys Ala Leu Pro Ala
225                 230                 235                 240

Pro Ile Glu Lys Thr Ile Ser Lys Ala Lys Gly Gln Pro Arg Glu Pro
                245                 250                 255

Gln Val Tyr Thr Leu Pro Pro Ser Arg Asp Glu Leu Thr Lys Asn Gln
            260                 265                 270

Val Ser Leu Thr Cys Leu Val Lys Gly Phe Tyr Pro Ser Asp Ile Ala
        275                 280                 285

Val Glu Trp Glu Ser Asn Gly Gln Pro Glu Asn Asn Tyr Lys Thr Thr
    290                 295                 300

Pro Pro Val Leu Asp Ser Asp Gly Ser Phe Phe Leu Tyr Ser Lys Leu
305                 310                 315                 320

Thr Val Asp Lys Ser Arg Trp Gln Gln Gly Asn Val Phe Ser Cys Ser
                325                 330                 335

Val Met His Glu Ala Leu His Asn His Tyr Thr Gln Lys Ser Leu Ser
            340                 345                 350

Leu Ser Pro Gly
        355

<210>  55
<211>  356
<212>  PRT
<213>  Artificial Sequence

<220>
<223>  Anti-PDL1-1D7V3-Fc

<220>
<221>  CHAIN
<222>  (1)..(356)

<400>  55

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Ala Phe Leu Thr Tyr
            20                  25                  30

Ala Met Gly Trp Phe Arg Gln Ala Pro Gly Lys Gly Leu Glu Phe Val
        35                  40                  45

Ala Ala Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Val Ser Gly Ala Thr Arg Asp His Leu Met Thr Thr Tyr
            100                 105                 110

Asp Tyr Trp Gly Gln Gly Thr Leu Val Thr Val Ser Ser Glu Pro Lys
        115                 120                 125

Ser Cys Asp Lys Thr His Thr Cys Pro Pro Cys Pro Ala Pro Glu Leu
    130                 135                 140

Leu Gly Gly Pro Ser Val Phe Leu Phe Pro Pro Lys Pro Lys Asp Thr
145                 150                 155                 160

Leu Met Ile Ser Arg Thr Pro Glu Val Thr Cys Val Val Val Asp Val
                165                 170                 175

Ser His Glu Asp Pro Glu Val Lys Phe Asn Trp Tyr Val Asp Gly Val
            180                 185                 190

Glu Val His Asn Ala Lys Thr Lys Pro Arg Glu Glu Gln Tyr Asn Ser
        195                 200                 205

Thr Tyr Arg Val Val Ser Val Leu Thr Val Leu His Gln Asp Trp Leu
    210                 215                 220

Asn Gly Lys Glu Tyr Lys Cys Lys Val Ser Asn Lys Ala Leu Pro Ala
225                 230                 235                 240

Pro Ile Glu Lys Thr Ile Ser Lys Ala Lys Gly Gln Pro Arg Glu Pro
                245                 250                 255

Gln Val Tyr Thr Leu Pro Pro Ser Arg Asp Glu Leu Thr Lys Asn Gln
            260                 265                 270

Val Ser Leu Thr Cys Leu Val Lys Gly Phe Tyr Pro Ser Asp Ile Ala
        275                 280                 285

Val Glu Trp Glu Ser Asn Gly Gln Pro Glu Asn Asn Tyr Lys Thr Thr
    290                 295                 300

Pro Pro Val Leu Asp Ser Asp Gly Ser Phe Phe Leu Tyr Ser Lys Leu
305                 310                 315                 320

Thr Val Asp Lys Ser Arg Trp Gln Gln Gly Asn Val Phe Ser Cys Ser
                325                 330                 335

Val Met His Glu Ala Leu His Asn His Tyr Thr Gln Lys Ser Leu Ser
            340                 345                 350

Leu Ser Pro Gly
        355

<210>  56
<211>  355
<212>  PRT
<213>  Artificial Sequence

<220>
<223>  Anti-PDL1-1G10V3-Fc


<220>
<221>  CHAIN
<222>  (1)..(355)

<400>  56

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Pro Phe Ile Thr Tyr
            20                  25                  30

Ala Met Gly Trp Phe Arg Gln Ala Pro Gly Lys Gly Leu Glu Phe Val
        35                  40                  45

Ala Ala Ile Asn Trp Ser Gly Ser Ile Thr Ala Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Lys Arg Thr Ala Val Ala Pro Arg Ala Leu Asn Asp Tyr Asp
            100                 105                 110

Phe Trp Gly Gln Gly Thr Leu Val Thr Val Ser Ser Glu Pro Lys Ser
        115                 120                 125

Cys Asp Lys Thr His Thr Cys Pro Pro Cys Pro Ala Pro Glu Leu Leu
    130                 135                 140

Gly Gly Pro Ser Val Phe Leu Phe Pro Pro Lys Pro Lys Asp Thr Leu
145                 150                 155                 160

Met Ile Ser Arg Thr Pro Glu Val Thr Cys Val Val Val Asp Val Ser
                165                 170                 175

His Glu Asp Pro Glu Val Lys Phe Asn Trp Tyr Val Asp Gly Val Glu
            180                 185                 190

Val His Asn Ala Lys Thr Lys Pro Arg Glu Glu Gln Tyr Asn Ser Thr
        195                 200                 205

Tyr Arg Val Val Ser Val Leu Thr Val Leu His Gln Asp Trp Leu Asn
    210                 215                 220

Gly Lys Glu Tyr Lys Cys Lys Val Ser Asn Lys Ala Leu Pro Ala Pro
225                 230                 235                 240

Ile Glu Lys Thr Ile Ser Lys Ala Lys Gly Gln Pro Arg Glu Pro Gln
                245                 250                 255

Val Tyr Thr Leu Pro Pro Ser Arg Asp Glu Leu Thr Lys Asn Gln Val
            260                 265                 270

Ser Leu Thr Cys Leu Val Lys Gly Phe Tyr Pro Ser Asp Ile Ala Val
        275                 280                 285

Glu Trp Glu Ser Asn Gly Gln Pro Glu Asn Asn Tyr Lys Thr Thr Pro
    290                 295                 300

Pro Val Leu Asp Ser Asp Gly Ser Phe Phe Leu Tyr Ser Lys Leu Thr
305                 310                 315                 320

Val Asp Lys Ser Arg Trp Gln Gln Gly Asn Val Phe Ser Cys Ser Val
                325                 330                 335

Met His Glu Ala Leu His Asn His Tyr Thr Gln Lys Ser Leu Ser Leu
            340                 345                 350

Ser Pro Gly
        355

<210>  57
<211>  356
<212>  PRT
<213>  Artificial Sequence

<220>
<223>  Anti-PDL1-5A8V3-Fc

<220>
<221>  PEPTIDE
<222>  (1)..(356)

<400>  57

Gln Val Gln Leu Val Glu Ser Gly Gly Gly Leu Val Gln Pro Gly Gly
1               5                   10                  15

Ser Leu Arg Leu Ser Cys Ala Ala Ser Gly Gly Thr Phe Ile Thr Tyr
            20                  25                  30

Ala Met Gly Trp Phe Arg Gln Ala Pro Gly Lys Gly Leu Glu Phe Val
        35                  40                  45

Ala Gly Ile Ser Trp Ser Gly Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Lys Ile Ser Gly Ala Thr Arg Glu His Leu Arg Thr Ser Tyr
            100                 105                 110

Asp Tyr Trp Gly Gln Gly Thr Leu Val Thr Val Ser Ser Glu Pro Lys
        115                 120                 125

Ser Cys Asp Lys Thr His Thr Cys Pro Pro Cys Pro Ala Pro Glu Leu
    130                 135                 140

Leu Gly Gly Pro Ser Val Phe Leu Phe Pro Pro Lys Pro Lys Asp Thr
145                 150                 155                 160

Leu Met Ile Ser Arg Thr Pro Glu Val Thr Cys Val Val Val Asp Val
                165                 170                 175

Ser His Glu Asp Pro Glu Val Lys Phe Asn Trp Tyr Val Asp Gly Val
            180                 185                 190

Glu Val His Asn Ala Lys Thr Lys Pro Arg Glu Glu Gln Tyr Asn Ser
        195                 200                 205

Thr Tyr Arg Val Val Ser Val Leu Thr Val Leu His Gln Asp Trp Leu
    210                 215                 220

Asn Gly Lys Glu Tyr Lys Cys Lys Val Ser Asn Lys Ala Leu Pro Ala
225                 230                 235                 240

Pro Ile Glu Lys Thr Ile Ser Lys Ala Lys Gly Gln Pro Arg Glu Pro
                245                 250                 255

Gln Val Tyr Thr Leu Pro Pro Ser Arg Asp Glu Leu Thr Lys Asn Gln
            260                 265                 270

Val Ser Leu Thr Cys Leu Val Lys Gly Phe Tyr Pro Ser Asp Ile Ala
        275                 280                 285

Val Glu Trp Glu Ser Asn Gly Gln Pro Glu Asn Asn Tyr Lys Thr Thr
    290                 295                 300

Pro Pro Val Leu Asp Ser Asp Gly Ser Phe Phe Leu Tyr Ser Lys Leu
305                 310                 315                 320

Thr Val Asp Lys Ser Arg Trp Gln Gln Gly Asn Val Phe Ser Cys Ser
                325                 330                 335

Val Met His Glu Ala Leu His Asn His Tyr Thr Gln Lys Ser Leu Ser
            340                 345                 350

Leu Ser Pro Gly
        355

<210>  58
<211>  354
<212>  PRT
<213>  Artificial Sequence

<220>
<223>  Anti-PDL1-6F1V3-Fc


<220>
<221>  PEPTIDE
<222>  (1)..(354)

<400>  58

Gln Val Gln Leu Val Glu Ser Gly Gly Gly Leu Val Gln Pro Gly Gly
1               5                   10                  15

Ser Leu Arg Leu Ser Cys Ala Ala Ser Gly Arg Thr Phe Ser Thr His
            20                  25                  30

Ser Met Gly Trp Phe Arg Gln Ala Pro Gly Lys Gly Leu Glu Phe Val
        35                  40                  45

Ala Ala Ile Asn Trp Asn Gly Asp Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ala Arg Pro Gly Ala Pro Ser Thr Ile Val Ala Met Leu Asp Tyr
            100                 105                 110

Trp Gly Gln Gly Thr Leu Val Thr Val Ser Ser Glu Pro Lys Ser Cys
        115                 120                 125

Asp Lys Thr His Thr Cys Pro Pro Cys Pro Ala Pro Glu Leu Leu Gly
    130                 135                 140

Gly Pro Ser Val Phe Leu Phe Pro Pro Lys Pro Lys Asp Thr Leu Met
145                 150                 155                 160

Ile Ser Arg Thr Pro Glu Val Thr Cys Val Val Val Asp Val Ser His
                165                 170                 175

Glu Asp Pro Glu Val Lys Phe Asn Trp Tyr Val Asp Gly Val Glu Val
            180                 185                 190

His Asn Ala Lys Thr Lys Pro Arg Glu Glu Gln Tyr Asn Ser Thr Tyr
        195                 200                 205

Arg Val Val Ser Val Leu Thr Val Leu His Gln Asp Trp Leu Asn Gly
    210                 215                 220

Lys Glu Tyr Lys Cys Lys Val Ser Asn Lys Ala Leu Pro Ala Pro Ile
225                 230                 235                 240

Glu Lys Thr Ile Ser Lys Ala Lys Gly Gln Pro Arg Glu Pro Gln Val
                245                 250                 255

Tyr Thr Leu Pro Pro Ser Arg Asp Glu Leu Thr Lys Asn Gln Val Ser
            260                 265                 270

Leu Thr Cys Leu Val Lys Gly Phe Tyr Pro Ser Asp Ile Ala Val Glu
        275                 280                 285

Trp Glu Ser Asn Gly Gln Pro Glu Asn Asn Tyr Lys Thr Thr Pro Pro
    290                 295                 300

Val Leu Asp Ser Asp Gly Ser Phe Phe Leu Tyr Ser Lys Leu Thr Val
305                 310                 315                 320

Asp Lys Ser Arg Trp Gln Gln Gly Asn Val Phe Ser Cys Ser Val Met
                325                 330                 335

His Glu Ala Leu His Asn His Tyr Thr Gln Lys Ser Leu Ser Leu Ser

340                 345                 350

Pro Gly

<210>  59
<211>  17
<212>  PRT
<213>  Artificial Sequence

<220>
<223>  hu1A7V3 FR2

<220>
<221>  PEPTIDE
<222>  (1)..(17)

<400>  59

Ile Asp Trp Tyr Arg Gln Ala Pro Gly Lys Gly Leu Glu Leu Val Ala
1               5                   10                  15

Val

<210>  60
<211>  17
<212>  PRT
<213>  Artificial Sequence

<220>
<223>  hu1D1V3/ hu5A8V3 FR2

<220>
<221>  PEPTIDE
<222>  (1)..(17)

<400>  60

Met Gly Trp Phe Arg Gln Ala Pro Gly Lys Gly Leu Glu Phe Val Ala
1               5                   10                  15

Gly

<210>  61
<211>  17
<212>  PRT
<213>  Artificial Sequence

<220>
<223>   hu1D7V3/ hu1G10V3/ hu6F1V3 FR2

<220>

<221>  PEPTIDE
<222>  (1)..(17)

<400>  61

Met Gly Trp Phe Arg Gln Ala Pro Gly Lys Gly Leu Glu Phe Val Ala
1               5                   10                  15

Ala

<210>  62
<211>  38
<212>  PRT
<213>  Artificial Sequence

<220>
<223>  hu1A7V3 FR3

<220>
<221>  PEPTIDE
<222>  (1)..(38)

<400>  62

Ser Tyr Ala Asp Ser Val Lys Gly Arg Phe Thr Ile Ser Arg Asp Asn
1               5                   10                  15

Ser Lys Asn Thr Leu Tyr Leu Gln Met Asn Ser Leu Arg Ala Glu Asp
            20                  25                  30

Thr Ala Val Tyr Tyr Cys
        35

<210>  63
<211>  38
<212>  PRT
<213>  Artificial Sequence

<220>
<223>  hu1D1V3/ hu1D7V3/ hu5A8V3/ hu6F1V3 FR3

<220>
<221>  PEPTIDE
<222>  (1)..(38)

<400>  63

Tyr Tyr Ala Asp Ser Val Lys Gly Arg Phe Thr Ile Ser Arg Asp Asn
1               5                   10                  15

Ser Lys Asn Thr Leu Tyr Leu Gln Met Asn Ser Leu Arg Ala Glu Asp
            20                  25                  30

Thr Ala Val Tyr Tyr Cys
        35

<210>  64
<211>  38
<212>  PRT
<213>  Artificial Sequence

<220>
<223>  hu1G10V3 FR3

<220>
<221>  PEPTIDE
<222>  (1)..(38)

<400>  64

Ala Tyr Ala Asp Ser Val Lys Gly Arg Phe Thr Ile Ser Arg Asp Asn
1               5                   10                  15

Ser Lys Asn Thr Leu Tyr Leu Gln Met Asn Ser Leu Arg Ala Glu Asp
            20                  25                  30

Thr Ala Val Tyr Tyr Cys
        35

<210>  65
<211>  11
<212>  PRT
<213>  Artificial Sequence

<220>
<223>  hu1A7V3/ hu1D1V3/ hu1D7V3/ hu1G10V3/ hu5A8V3/ hu6F1V3 FR4

<220>
<221>  PEPTIDE
<222>  (1)..(11)

<400>  65

Trp Gly Gln Gly Thr Leu Val Thr Val Ser Ser
1               5                   10

<210>  66
<211>  21
<212>  PRT
<213>  Artificial Sequence

<220>
<223>  PDL1-56 dAb

<220>
<221>  PEPTIDE
<222>  (1)..(21)

<400>  66

Ala Ala Asp Ser Phe Glu Asp Pro Thr Cys Thr Leu Val Thr Ser Ser
1               5                   10                  15

Gly Ala Phe Gln Tyr
            20

<210>  67
<211>  26
<212>  PRT
<213>  Artificial Sequence

<220>
<223>  Innovent Nb

<220>
<221>  PEPTIDE
<222>  (1)..(26)

<400>  67

Ala Ala Pro Lys Val Gly Leu Gly Pro Arg Thr Ala Leu Gly His Leu
1               5                   10                  15

Ala Phe Met Thr Leu Pro Ala Leu Asn Tyr
            20                  25

<210>  68
<211>  19
<212>  PRT
<213>  Artificial Sequence

<220>
<223>  Nb 43

<220>
<221>  PEPTIDE
<222>  (1)..(19)

<400>  68

Ala Ala Asp Asp Asp Tyr Tyr Ala Phe Leu Ser Arg Gly Ala Arg Asp
1               5                   10                  15

Phe Arg Tyr

## Description

### Technical Field

The present disclosure relates to the field of biotechnology, and particularly to an anti-human PD-L1 single-domain antibody and use thereof.

### Background

Programmed death 1 ligand 1 (PD-L1) was originally cloned as a member of the B7 protein family (referred to as B7-H1) (Dong et al., 1999 Nature Med 5:1365). PD-L1 is a type I transmembrane protein with a total of 290 amino acids, and including one IgV-like domain, one IgC-like domain, one transmembrane hydrophobic domain, and one intracellular domain consisting of 30 amino acids. PD-L1 is widely expressed on the surface of various immune cells, epithelial cells and tumor cells. PD-L1 binds to the programmed death-1 (PD-1) receptor and activates the negative regulation signaling pathway, to inhibit T cell activation or induce T cell apoptosis, thereby suppressing the immune responses (Freeman et al., 2000J Exp Med 192:1027). In the development of tumors, cancer cells induce T cell apoptosis by up-regulating PD-L1 expression, to escape from elimination by immune system, thus leading to the disease progression. In recent years, monoclonal antibody drugs targeting PD-1/PD-L1 are used to block the binding of PD-1/PD-L1, thus promoting the activation and proliferation of T cells in vivo, and killing tumor cells. They are used in the treatment of various tumors, such as melanoma, lymphoma, bladder cancer, non-small cell lung cancer, head and neck cancer, and colon cancer, etc., with remarkable therapeutic effect. Therefore, the PD1/PD-L1 pathway is an important target for antitumor drugs. At present, antibody drugs that inhibit the PD1/PD-L1 pathway have achieved great success in clinic. For example, Nivolumab developed by Bristol-Myers Squibb, MK-3475 developed by Merck and MPDL3280A developed by Roche are marketed successively. The antibody drugs targeting the PD1/PD-L1 pathway will become the most potential direction in the tumor treatment market.

The successful use of monoclonal antibodies in the cancer detection and biological targeted therapy leads to an innovation in the cancer treatment. However, the traditional monoclonal antibodies (about 150kD) have a large molecular weight, and thus have difficulty in penetrating the tissues, resulting in low effective concentration in the tumor area and poor therapeutic effect. Traditional antibodies are highly immunogenic, and it is difficult for the modified antibodies to achieve previous affinity. Moreover, fully humanized traditional antibodies have disadvantages such as long development period, high production cost, insufficient stability and others that limit their universal use in clinic. The subsequent single-domain antibodies include the smallest functional antigen-binding fragment derived from a heavy chain antibody in adult camelid, which has high stability and high affinity for antigen binding. Compared with conventional antibodies, single-domain antibodies have many unique properties: 1) The sequences encoded by single-domain antibodies are highly homologous to human VH families 3 and 4, making them less immunogenic; 2) The single-domain antibodies have a small molecular weight that is only about 15kDa, and thus are simple in structure, tend to express at a high level in microorganisms, and can be easy to be purified. The unique properties and low cost of single-domain antibodies greatly expand their scope of application, showing the significance in the treatment and diagnosis of diseases. However, in the prior art, it is difficult for those skilled in the art to screen single-domain antibodies, so single-domain antibodies with high affinity, high specificity and promising prospect in industrialization are difficult to be obtained. It is far more difficult to screen antibodies that have only three complementarity determining regions (CDRs) to bind antigens than antibodies that have six CDRs to bind antigens. Herein the current antibodies that are commercialized or under preclinical research are mostly monoclonal antibodies with large molecular weight. Moreover, there are also some problems in the use of single-domain antibodies obtained in the art, for example, the immunogenicity (ADA) caused by their special structures. TAS266 is an anti-DR5 receptor single-domain antibody, the development of which is interrupted due to severe hepatotoxicity in Phase I clinical trial. Further studies show that the hepatotoxicity is closely related to the presence of ADA in subjects (Papadopoulos, K.P., Isaacs, R., Bilic, S. et al. Cancer Chemother Pharmacol (2015) 75: 887.). The clinical studies of another anti-TNFR1 VHH antibody (GSK1995057) is also interrupted in phase I clinical trial, due to cytokine storm caused by the presence of ADA in subjects (Journal of Clinical Immunology, 2013, Volume 33, Number 7, Page 1192 M. C. Holland, J. U. Wurthner, P. J. Morley). Single-heavy chain antibodies (VHHs) are somewhat different in framework regions and CDRs from the conventional human antibodies, to achieve high affinity and stability in the absence of light chains. In particular, the structure of CDR3 tends to be more different. In most cases, the amino acid composition and length of the CDR3 region in a high-affinity VHH obtained by screening are quite different from those of human antibody sequences (Nature (1993) 363:446-8; Protein Eng (1994) 7:1129-35.; Mol Immunol (1997) 34:1121-31). For example, the CDR3 region of a high-affinity VHH tends to be longer, in which Cys is also often present, and even disulfide bonds are formed (Protein Engineering, Design & Selection, 24(9): 727tein, 2011). The CDR1 and CDR3 (according to IMGT numbering) of the PDL1-56 sequence as shown in SEQ ID NO: 28 in WO2017/020801A1 each have one Cys, and the two cysteines significantly increase the chance of disulfide scrambling, leading to misfolding; and the pair of disulfide bonds formed by the 2 cysteines cause the difference from human antibodies to increase, such that the risk of immunogenicity may be increased. It is well known that as the difference from the human sequence increases, or the degree of humanization decreases, the potential immunogenicity increases. When they are used in the human body, the human body is more likely to produce anti-VHH antibodies. The immunogenicity caused by a biological drug often determines its efficacy and safety in clinical use, and thus is a key to its successful development for clinical use. An important contribution of the present disclosure is that some high-affinity VHH sequences that are very close in structure to the human antibodies are screened.

### Summary

In view of the shortcomings in the prior art described above, an object of the present disclosure is to provide an anti-human PD-L1 single-domain antibody (nanobody), to solve the problems existing in the prior art.

To achieve the above and other related objects, in a first aspect of the present disclosure, an anti-PD-L1 single-domain antibody is provided, which has a complementarity determining region (CDR) including CDR1-CDR3 having amino acid sequences shown below: (1) CDR1 as shown in SEQ ID NO: 6, CDR2 as shown in SEQ ID NO: 15, and CDR3 as shown in SEQ ID NO: 25; or (2) CDR1 as shown in SEQ ID NO: 7, CDR2 as shown in SEQ ID NO: 16, and CDR3 as shown in SEQ ID NO: 26; or (3) CDR1 as shown in SEQ ID NO: 8, CDR2 as shown in SEQ ID NO: 16, and CDR3 as shown in SEQ ID NO: 27; or (4) CDR1 as shown in SEQ ID NO: 9, CDR2 as shown in SEQ ID NO: 17, and CDR3 as shown in SEQ ID NO: 28; or (5) CDR1 as shown in SEQ ID NO: 7, CDR2 as shown in SEQ ID NO: 16, and CDR3 as shown in SEQ ID NO: 29; or (6) CDR1 as shown in SEQ ID NO: 10, CDR2 as shown in SEQ ID NO: 18, and CDR3 as shown in SEQ ID NO: 30.

In a preferred embodiment, the anti-PD-L1 single-domain antibody also has a framework region (FR), including FR1-FR4 having amino acid sequences shown below: (1') FR1 as shown in SEQ ID NO: 1, FR2 as shown in SEQ ID NO: 11, FR3 as shown in SEQ ID NO: 19, and FR4 as shown in SEQ ID NO: 31; or (2') FR1 as shown in SEQ ID NO: 2, FR2 as shown in SEQ ID NO: 12, FR3 as shown in SEQ ID NO: 20, and FR4 as shown in SEQ ID NO: 31; or (3') FR1 as shown in SEQ ID NO: 3, FR2 as shown in SEQ ID NO: 13, FR3 as shown in SEQ ID NO: 21, and FR4 as shown in SEQ ID NO: 31; or (4') FR1 as shown in SEQ ID NO: 4, FR2 as shown in SEQ ID NO: 13, FR3 as shown in SEQ ID NO: 22, and FR4 as shown in SEQ ID NO: 31; or (5') FR1 as shown in SEQ ID NO: 5, FR2 as shown in SEQ ID NO: 12, FR3 as shown in SEQ ID NO: 23, and FR4 as shown in SEQ ID NO: 32; or (6') FR1 as shown in SEQ ID NO: 5, FR2 as shown in SEQ ID NO: 14, FR3 as shown in SEQ ID NO: 24, and FR4 as shown in SEQ ID NO: 31.

In another preferred embodiment, the anti-PD-L1 single-domain antibody includes: (a) a single-domain antibody having an amino acid sequence as shown in any one of SEQ ID NOs: 33-38; or (b) a single-domain antibody having an amino acid sequence identity of 80% or above (e.g. 85%, 90%, 93%, 95%, 97% or 99% or more) with any one of SEQ ID NOs: 33-38 and having the functions of the (a) single-domain antibody.

In another preferred embodiment, the anti-PD-L1 single-domain antibody is a humanized antibody. Preferably, the framework region (FR) includes FR1-FR4 having amino acid sequences selected from the group consisting of: FR1 as shown in SEQ ID NO: 3; FR2 as shown in SEQ ID NOs: 59-61; FR3 as shown in SEQ ID NOs: 62-64; and FR4 as shown in SEQ ID NO: 65.

In another preferred embodiment, FR1 as shown in SEQ ID NO: 3 is used as FR1 in the single-domain antibody of any one of (1)-(6);

In another preferred embodiment, FR2 as shown in SEQ ID NO: 59 is used as FR2 in the single-domain antibody of (1); FR2 as shown in SEQ ID NO: 60 is used as FR2 in the single-domain antibody of (2) or (5); and FR2 as shown in SEQ ID NO: 61 is used as FR2 in the single-domain antibody of (3), (4), or (6).

In another preferred embodiment, FR3 as shown in SEQ ID NO: 62 is used as FR3 in the single-domain antibody of (1); FR3 as shown in SEQ ID NO: 63 is used as FR3 in the single-domain antibody of (2), (3), (5), or (6); and FR3 as shown in SEQ ID NO: 64 is used as FR3 in the single-domain antibody of (4).

In another preferred embodiment, FR4 as shown in SEQ ID NO: 65 is used as FR4 in the single-domain antibody of any one of (1)-(6).

In another preferred embodiment, the anti-PD-L1 single-domain antibody is a humanized antibody, which includes: (i) a single-domain antibody having an amino acid sequence as shown in any one of SEQ ID NO: 39-44; or (ii) a single-domain antibody having an amino acid sequence identity of 80% or above (e.g. 85%, 90%, 93%, 95%, 97% or 99% or more) with any one of SEQ ID NOs: 39-44 and having the functions of the (i) single-domain antibody.

In another aspect of the present disclosure, there is provided a fusion protein including: a first domain, which is a single-domain antibody as described above; and a second domain, having the effect of extending the in-vivo half-life and/or binding to the effector cells.

In a preferred embodiment, the second domain includes (but not limited to) a Fc region of an immunoglobulin, preferably a Fc region of a human immunoglobulin; and/or serum albumin (such as human HSA) or a fragment thereof, a domain that binds to serum albumin (e.g., anti-serum albumin antibodies, including single-domain antibodies), polyethylene glycol, a polyethylene glycol-liposome complex, or a combination thereof; and/or a molecule that has binding affinity to surface molecules of T cells and/or are capable of binding to surface molecules present on T cells; preferably, the surface molecule includes, but not limited to, CD3.

In another preferred embodiment, the Fc region of a human immunoglobulin includes a mutation for altering the Fc-mediated effector functions, where the effector functions include CDC activity, ADCC activity, ADCP activity, or a combination thereof.

In another preferred embodiment, the immunoglobulin is selected from IgG, IgA1, IgA2, IgD, IgE, IgM, or a combination thereof. IgG is selected from IgG1, IgG2, IgG3, or IgG4 isotypes, or a combination thereof.

In another preferred embodiment, the amino acid sequence of the Fc region of an immunoglobulin is one selected from SEQ ID NOs: 45-48.

In another preferred embodiment, a linker peptide is included which connects the first domain and the second domain; and the linker peptide is preferably selected from a flexible polypeptide chain consisting of alanine and/or serine and/or glycine, and has a length of preferably 3-30 amino acids.

In another aspect of the present disclosure, an isolated polynucleotide is provided, which encodes any single-domain antibody described above, or any fusion protein described above.

In another aspect of the present disclosure, a construct is provided, which includes an isolated polynucleotide as described above.

In another aspect of the present disclosure, an antibody expression system is provided, which includes a construct described above or has a genome integrated with an exogenous polynucleotide described above; and preferably, the expression system is a cell expression system.

In another preferred embodiment, under conditions suitable for expressing the antibody, the antibody expression system is used to express the antibody or fusion protein; and preferably, the antibody or fusion protein is further purified and isolated.

In another aspect of the present disclosure, an immunoconjugate is provided, which includes: (A) any single-domain antibody described above, or any fusion protein described above; and (B) a functional molecule linked to (A) (including, but not limited to, covalent linkage, conjugation, attachment, and adsorption).

In another preferred embodiment, (B) includes, but not limited to, cytotoxins, radioisotopes, biologically active proteins, molecules targeting tumor surface marker, tumor -suppressing molecules, molecules or detectable markers that target surface markers of immune cells, extracellular hinge regions, transmembrane regions and intracellular signaling regions based on chimeric antigen receptor technology (CAR), or a combination thereof.

In another preferred embodiment, the molecule targeting tumor surface marker is an antibody or ligand that binds to the tumor surface marker; or the tumor-suppressing molecule is an anti-tumor cytokine or anti-tumor toxin.

In another preferred embodiment, the cytokine includes, for example, but not limited to, IL-12, IL-15, IFN-beta, and TNF-alpha.

In another preferred embodiment, the detectable marker includes, for example, but not limited to: fluorescent markers, and developing markers.

In another preferred embodiment, the antibody binding to tumor surface marker is an antibody that recognizes other antigens than PD-L1, where the other antigens include, but not limited to, EGFR, EGFRvIII, mesothelin, HER2, EphA2, Her3, EpCAM, MUC1, MUC16, CEA, Claudin 18.2, folate receptor, Claudin 6, WT1, NY-ESO-1, MAGE 3, ASGPR1 or CDH16.

In another preferred embodiment, the intracellular signaling region includes, for example, but is not limited to, the intracellular signaling region of CD3ζ chain, FcεRlγ tyrosine-based activation motif, costimulatory signal molecule CD27, CD28, CD137, CD134, MyD88, CD40, and others. The transmembrane region includes, for example, but not limited to: the transmembrane region of CD8 or CD28.

In another aspect of the present disclosure, a pharmaceutical composition (including a diagnostic composition, such as a diagnostic reagent) is provided. The pharmaceutical composition includes any single-domain antibody described above, or any fusion protein described above, or any immunoconjugate described above.

In another preferred embodiment, the pharmaceutical composition also includes a pharmaceutically acceptable carrier.

In another aspect of the present disclosure, use of the single-domain antibody, the fusion protein, the immunoconjugate, or the pharmaceutical composition in the preparation of formulations, kits, or pharmaceutical pack for the diagnosis, treatment or prevention of cancers is provided.

In another preferred embodiment, the cancers include: lung cancer, melanoma, gastric cancer, ovarian cancer, colon cancer, liver cancer, kidney cancer, bladder cancer, breast cancer, classic Hodgkin lymphoma, hematological malignancies, head and neck cancer or nasopharyngeal cancer, or a combination thereof.

In another aspect of the present disclosure, use of the single-domain antibody, the fusion protein, the immunoconjugate, or the pharmaceutical composition in preparation of formulations, kits, or pharmaceutical packs for the treatment or prevention of infectious diseases or chronic inflammatory diseases is provided.

In another aspect of the present disclosure, a kit or pharmaceutical pack is provided, which includes the single-domain antibody, the fusion protein, the immunoconjugate, or the pharmaceutical composition.

Other aspects of the present disclosure are apparent to those skilled in the art because of the content of the present disclosure.

### Brief Description of the Drawings

Fig. 1 shows a curve of Anti-PDL1-Fc fusion protein according to the present disclosure blocking PD-L1/PD-1 interaction.
Fig. 2 shows the effect of the Anti-PDL1-Fc fusion protein according to the present disclosure on the immune response of Jurkat cells detected by a luciferase detection kit.
Fig. 3 shows the effect of the Anti-PDL1-Fc fusion protein according to the present disclosure on the IL-2 secretion by mixed lymphocytes.
Fig. 4 shows the inhibitory effect of Anti-PDL1-Fc fusion protein according to the present disclosure on tumor growth.

### Detailed Description of the Preferred Embodiments

After extensive screening and in-depth research, the present inventors disclose a class of specific anti-PD-L1 single-domain antibodies that block PD-L1/PD-1 interaction; humanized antibodies and fusion proteins prepared based on the single-domain antibodies; and use thereof.

### Terms

As used herein, the terms "programmed death ligand 1", "PD-ligand 1", "PD-L1", "PDL1", "B7 homologue 1", "B7-H1", and "CD274" are used interchangeably, and include variants, isotypes, species homologues of human PD-L1 and analogs with at least one common epitope of PD-L1.

Regardless of a heavy-chain antibody or a conventional 4-chain antibody, the term "antibody" or "immunoglobulin" herein is a general term to include a full-length antibody, a single chain, and a moiety, domain or fragment (including, but not limited to, an antigen binding domain or fragment, e.g. VHH domain or VH/VL domain) thereof. Moreover, as used herein, the term "sequence" (e.g. in the terms "immunoglobulin sequence", "antibody sequence", "single variable domain sequence", "VHH sequence" or "protein sequence" etc.) is generally understood to include both a relevant amino acid sequence, and a nucleic acid sequence or nucleotide sequence encoding the sequence, unless otherwise specifically defined herein.

The term "monoclonal antibody" refers to a preparation of antibody molecules consisting of a single molecule. Monoclonal antibodies show a single binding specificity and affinity for a specific epitope.

The term "domain" (of a polypeptide or protein) refers to a folded protein structure that is able to maintain a tertiary structure independently of other moieties of the protein. Generally, the domain is responsible for an individual functional property of the protein, and can be, in many cases, added, removed or transferred to other proteins without compromising the functions of other moieties and/or domains of the protein.

The term "single domain antibody (VHH)" refers to a heavy chain variable region of a cloned antibody, and a single-domain antibody (VHH) constructed to consist of only one heavy chain variable region is the smallest antigen-binding fragment with complete functionality. Usually, an antibody with natural deletion of light chain and heavy chain constant region 1 (CH1) is derived from alpaca immune serum, and then a heavy chain variable region of the antibody is cloned, to construct a single-domain antibody (VHH) consisting of only one heavy chain variable region.

The term "single domain antibody (VHH)" refers to an immunoglobulin domain including four "framework regions" that are referred to as "framework region1" or "FR1", "framework region 2" or "FR2", "framework region 3" or "FR3", and "framework region 4" or "FR4" in the art and hereinafter. The framework regions are spaced apart by three "complementarity determining regions" or "CDRs" that are referred to as "complementarity determining region 1" or "CDR1", "complementarity determining region 2" or "CDR2", and "complementarity determining region 3" or "CDR3" in the art and hereinafter. Therefore, the general structure or sequence of a single-domain antibody (VHH) can be expressed as FR1-CDR1-FR2-CDR2-FR3-CDR3-FR4. A single-domain antibody (VHH) confers an antigen binding specificity to the antibody due to the presence of an antigen-binding site.

The term "heavy-chain single-domain antibody", "VHH domain", "VHH", "VHH antibody fragment", "VHH antibody" and "nanobody" are used interchangeably.

The term "IMGT numbering system" is an integrated information system dedicated for immunoglobulins (IGs), T cell receptors (TCRs) and major histocompatibility complexes (MHCs) of humans and other vertebrates, that is, THE INTERNATIONAL IMMUNOGENETICS INFORMATION SYSTEM^{®} (Lafranc et al., 2003, Dev.Comp.Immunol. 27(1):55-77). Log on to IMGT (http://www.imgt.org/ IMGT_ vquest) to analyze the light and heavy chains of an antibody, so as to determine the framework regions (FRs) and complementarity determining regions CDRs) of the variable region. The "position" of CDRs within the structure of immunoglobulin variable domains is conserved between species, and exists in a structure called loop. By using a numbering system that aligns variable domain sequences according to structural features, CDRs and framework residues can be easily identified. The information can be used to graft and insert CDR residues of an immunoglobulin from one species into a receptor framework usually from a human antibody. Unless otherwise indicated, in the specification, claims and drawings, the anti-PD-L1 single-domain antibodies are numbered according to the IMGT numbering system, to determine the CDR and FR regions.

The term "humanized antibody" refers to a molecule having an antigen-binding site substantially derived from an immunoglobulin from a non-human species, where the rest of the immunoglobulin structure of the molecule is based on the structure and/or sequence of a human immunoglobulin. The antigen binding site may include a complete variable domain fused to a constant domain, or only include a complementarity determining region (CDR) grafted into an appropriate framework region in the variable domain. The antigen binding site can be wild-type, or modified by one or more amino acid substitutions. For example, modifications are made to allow the antibody to be more similar to human immunoglobulins. Some forms of humanized antibodies retain all CDR sequences (e.g., a humanized single-domain antibody including all three CDRs from alpaca). Other forms have one or more CDRs that have been altered relative to the original antibody.

The term "protein aggregate" means that the protein structures are stabilized by non-covalent interactions and a disulfide linkage between two cysteine residues. The noncovalent interaction includes ionic interactions and weak van der Waals interactions. The ionic interaction is formed between anions and cations, and form a salt bridge, to stabilize the protein. It plays an important role in the secondary structure, for example, in the formation of alpha helices or beta sheets, and in the tertiary structure of proteins. The interactions between amino acid residues in a particular protein are very important in the final structure of that proteins. Commonly in the industrial preparation of active proteins in vitro or extracellularly, when for example, the pH, concentration, ionic strength or temperature changes in the environment where the active protein is present, noncovalent interactions between amino acids are changed, causing that the exposed hydrophobic moiety of one protein molecule may interact with the exposed hydrophobic moiety of other protein molecules, to form an amorphous aggregate, an oligomer and a high molecular weight aggregate. The aggregate affects the stability of active proteins. The in-vivo formation of aggregates easily triggers a clearance response by the immune system, which accelerates the drug clearance and may cause an immune risk to the body.

The "sequence identity" between two polypeptide sequences indicates the percentage of identical amino acids between the sequences. The "sequence similarity" indicates the percentage of identical or conservatively substituted amino acids among sequences. Methods for assessing the degree of sequence identity between amino acid sequences or nucleotide sequences are known to those skilled in the art. For example, the amino acid sequence identity is typically determined by sequence analysis software. For example, the identity can be determined using the BLAST program of the NCBI database.

An "effective amount" of an agent refers to an amount necessary to cause a physiological change in a cell or tissue to which it is administered.

A "therapeutically effective amount" of an agent, such as a pharmaceutical composition, refers to an amount effective for achieving the desired therapeutic or prophylactic result at a dosage level and for a period of time. A therapeutically effective amount of an agent can, for example, eliminate, reduce, delay, minimize or prevent the adverse effects of a disease.

An "individual" or "subject" is a mammal, including, but not limited to, domesticated animals (e.g. cows, sheep, cats, dogs and horses), primates (e.g. humans and non-human primates such as monkeys), rabbits and rodents (e.g. mice and rats). Preferably, the individual or subject is a human.

The term "pharmaceutical composition" refers to a preparation in a form that allows the biological activity of the active ingredient contained therein to be effective, and do not contain other ingredients that would be unacceptably toxic to subjects to whom the composition would be administered.

"Pharmaceutically acceptable carrier" refers to an ingredient other than the active ingredient in a pharmaceutical composition that is not toxic to the subject. The pharmaceutically acceptable carriers include, but are not limited to, buffers, excipients, stabilizers or preservatives.

The term "treating/preventing" (and its grammatical variants) refers to an attempt to alter the natural progress of a disease in a treated individual, and can be prevention or clinical intervention implemented during the course of clinical pathology. The desired effect of treatment includes, but is not limited to, preventing the occurrence or recurrence of diseases, alleviating the symptoms, relieving any direct or indirect pathological consequences of the disease, preventing the metastasis, slowing the rate of disease progression, improving or lessening the disease states, and relieving or improving the prognosis. In some embodiments, the antibody of the present disclosure is useful for delaying the development of a disease or delaying the progression of a disorder.

The term "detectable marker" refers to a marker that can be attached to an antibody, and used to determine the presence or amount of a specific target in a subject to be tested. The "detectable marker" may be, but is not limited to: an enzyme, a fluorescent label, a radionuclide, quantum dots, or colloidal gold, etc.; and more specifically, for example, horseradish peroxidase (HRP), alkaline phosphatase (AP), glucose oxidase, β~D-galactosidase, urease, hydrogen peroxidase, or glucoamylase.

In the present disclosure, the cancer or tumor includes, but is not limited to, lung cancer, melanoma, gastric cancer, ovarian cancer, colon cancer, liver cancer, kidney cancer, bladder cancer, breast cancer, classic Hodgkin lymphoma, hematological malignancies, head and neck cancer or nasopharyngeal cancer, or a combination thereof, and also metastases or complications thereof.

### Single-domain antibody

In a first aspect of the present disclosure, an anti-PD-L1 single-domain antibody is provided. Complementarity determining region (CDR) of the anti-PD-L1 single-domain antibody includes CDR1-CDR3 having amino acid sequences shown below: (1) CDR1 as shown in SEQ ID NO: 6, CDR2 as shown in SEQ ID NO: 15, and CDR3 as shown in SEQ ID NO: 25; or (2) CDR1 as shown in SEQ ID NO: 7, CDR2 as shown in SEQ ID NO: 16, and CDR3 as shown in SEQ ID NO: 26; or (3) CDR1 as shown in SEQ ID NO: 8, CDR2 as shown in SEQ ID NO: 16, and CDR3 as shown in SEQ ID NO: 27; or (4) CDR1 as shown in SEQ ID NO: 9, CDR2 as shown in SEQ ID NO: 17, and CDR3 as shown in SEQ ID NO: 28; or (5) CDR1 as shown in SEQ ID NO: 7, CDR2 as shown in SEQ ID NO: 16, and CDR3 as shown in SEQ ID NO: 29; or (6) CDR1 as shown in SEQ ID NO: 10, CDR2 as shown in SEQ ID NO: 18, and CDR3 as shown in SEQ ID NO: 30.

The anti-PD-L1 antigen single-domain antibody provided in the present disclosure may include a framework region FR. The framework region FR includes FR1-FR4 having amino acid sequences shown below: (1') FR1 as shown in SEQ ID NO: 1, FR2 as shown in SEQ ID NO: 11, FR3 as shown in SEQ ID NO: 19, and FR4 as shown in SEQ ID NO: 31; or (2') FR1 as shown in SEQ ID NO: 2, FR2 as shown in SEQ ID NO: 12, FR3 as shown in SEQ ID NO: 20, and FR4 as shown in SEQ ID NO: 31; or (3') FR1 as shown in SEQ ID NO: 3, FR2 as shown in SEQ ID NO: 13, FR3 as shown in SEQ ID NO: 21, and FR4 as shown in SEQ ID NO: 31; or (4') FR1 as shown in SEQ ID NO: 4, FR2 as shown in SEQ ID NO: 13, FR3 as shown in SEQ ID NO: 22, and FR4 as shown in SEQ ID NO: 31; or (5') FR1 as shown in SEQ ID NO: 5, FR2 as shown in SEQ ID NO: 12, FR3 as shown in SEQ ID NO: 23, and FR4 as shown in SEQ ID NO: 32; or (6') FR1 as shown in SEQ ID NO: 5, FR2 as shown in SEQ ID NO: 14, FR3 as shown in SEQ ID NO: 24, and FR4 as shown in SEQ ID NO: 31.

The antigen-binding property of the antibody is usually determined by three complementarity determining regions (CDRs), where the CDR regions and FR regions are arranged in order, and the FR regions do not directly participate in the binding reaction. These CDRs form a loop structure, and are spatially close to each other by means of the β-sheets formed by the FRs therebetween, to form the antigen-binding site of the antibody. The CDR regions are sequences of proteins of immunological interest, and the CDR regions of the antibody of the present disclosure are completely new.

The amino acid sequence of the anti-PD-L1 antigen single-domain antibody provided in the present disclosure may include: a) an amino acid sequence shown in one of SEQ ID NOs: 33-38; or, b) an amino acid sequence having 80% or more sequence identity to one of SEQ ID NO: 33-38, and having the functions of the amino acid sequence defined in a). Specifically, the amino acid sequence in b) specifically refers to a polypeptide fragment derived from the amino acid sequence as shown in one of SEQ ID NOs: 33-38 by the substitution, deletion or addition of one or more (specifically 1-50, 1-30, 1-20, 1-10, 1-5, or 1-3) amino acids, or by the addition or deletion of one or more (specifically 1-50, 1-30, 1-20, 1-10, 1-5, or 1-3) amino acids at the N- and/or C-terminus, and having the functions of a polypeptide fragment having an amino acid sequence as shown in one of SEQ ID NOs: 33-38, for example, the ability to specifically bind to PD-L1. The amino acid sequence as shown in b) may have at least 80%, 85%, 90%, 93%, 95%, 97%, or 99% sequence homology or sequence identity to one of SEQ ID NO: 33-38. The anti-PD-L1 single-domain antibody provided in the present disclosure can specifically bind to human PD-L1, block the interaction between PD-L1 and PD-1, increase the expression of IFN-γ and/or IL-2 in T lymphocytes, and inhibit the tumor growth.

The anti-PD-L1 single-domain antibody provided in the present disclosure can be a humanized antibody, and the humanized single-domain antibody can retain at least one of the functional properties of the antibody, for example, the ability to specifically bind to PD-L1 or block the interaction between PD-L1 and PD-1. On the basis of use of the specific complementarity determining regions in the present disclosure, humanized antibodies designed according to techniques in the art are all embraced in the present disclosure.

In a preferred embodiment of the present disclosure, a naturally occurring single-domain antibody from Camelidae is humanized by the present inventors, to further improve the drug safety, and effectively reduce the immunogenicity of the antibody. Fully humanized heavy chain antibodies often have the problem of protein aggregation caused by poor solubility, and thus fail to be used in actual clinical practice. Decreased solubility of the single-domain antibody due to humanization may attribute to their lack of a light chain that a natural monoclonal antibody paired with (Front Immunol. 2017 Nov 22;8:1603). The humanized antibody with modified framework region obtained in the present disclosure still maintains a high solubility, making it feasible for use in practical clinical application. In a specific embodiment of the present disclosure, the amino acid sequences of the anti-PD-L1 humanized single-domain antibody are as shown in SEQ ID NO: 39-44.

### Fusion protein

In a second aspect of the present disclosure, a fusion protein of an anti-PD-L1 single-domain antibody is provided, which includes a first domain of the single-domain antibody provided in the first aspect of the present disclosure and a second domain for prolonging the half-life in vivo and/or having the ability to bind to effector cells. The fusion protein can be a binding molecule, which can specifically bind to cells expressing PD-L1.

In the second domain, the fragment for extending the half-life in vivo may include serum albumin or a fragment thereof, polyethylene glycol, a domain binding to serum albumin (such as a single-domain antibody against serum albumin), or a polyethylene glycol-liposome complex, etc. In the second domain, the fragment capable of binding to effector cells may include an Fc region of an immunoglobulin molecule, etc., and preferably an Fc region of a human immunoglobulin molecule. The Fc region of a human immunoglobulin includes a mutation for altering the Fc-mediated effector functions, where the effector functions include CDC activity, ADCC activity, ADCP activity, or a combination thereof. The immunoglobulin is selected from IgG, IgA1, IgA2, IgD, IgE, IgM, or a combination thereof. IgG is specifically selected from IgG1, IgG2, IgG3, or IgG4 subtype, or a combination thereof. The Fc region of an immunoglobulin contained in the fusion protein of a single-domain antibody makes the fusion protein form a dimer, prolong the in vivo half-life of the fusion protein and increase the related activity mediated by Fc. In a specific embodiment of the present disclosure, the Fc region of an immunoglobulin can be the Fc region of human IgG1, and more specifically Fc sequence of wild-type IgG1. A mutation that alters Fc-mediated effector function can be introduced into the sequence, for example, a) a mutation that alters the Fc-mediated CDC activity; b) a mutation that alters the Fc-mediated ADCC activity; or a) a mutation that alters the Fc-mediated ADCP activity. Such mutations are described in: Leonard G Presta, Current Opinion in Immunology 2008, 20:460-470; Esohe E.Idusogie et al., J Immunol 2000, 164:4178-4184; RAPHAELA. CLYNES et al., Nature Medicine, 2000, Volume 6, Number 4:443-446; Paul R. Hinton et al., J Immunol, 2006, 176:346-356. In another specific embodiment of the present disclosure, the Fc region of the immunoglobulin has an amino acid sequence selected from SEQ ID NOs: 45-48.

In the fusion protein of the anti-PD-L1 single-domain antibody provided in the present disclosure, a linker peptide may be included, which connects the first domain and the second domain. The linker peptide may be a flexible polypeptide chain consisting of alanine (A) and/or serine (S) and/or glycine (G), having a length of 3-30 amino acids, preferably 3-9, 9-12, 12-16, 16-20 amino acids. In another specific embodiment of the present disclosure, the linker peptide may have a length of 8 or 15 amino acids.

### Isolated polynucleotide

In a third aspect of the present disclosure, an isolated polynucleotide is provided, which encodes the single-domain antibody provided in the first aspect of the present disclosure, or the fusion protein provided in the second aspect of the present disclosure. The polynucleotide may be RNA, DNA or cDNA, etc. Methods of providing the isolated polynucleotide are known to those skilled in the art, for example, preparation by automatic DNA synthesis and/or recombinant DNA technology, or isolation from a suitable natural source.

### Construct

In a fourth aspect of the present disclosure, a construct is provided. The construct includes the isolated polynucleotide provided in the third aspect of the present disclosure. A method of constructing the construct is known to those skilled in the art. For example, the construct can be obtained by in-vitro recombinant DNA technology, DNA synthesis technology, or in-vivo recombinant technology. More specifically, it can be constructed by inserting the isolated polynucleotide into a polyclonal site of an expression vector. The expression vector in the present disclosure generally refers to various commercially available expression vectors well known in the art, for example, bacterial plasmids, bacteriophages, yeast plasmids, plant cell-infected viruses, mammalian cell-infected viruses such as adenovirus, retrovirus or other vectors. The vector may also include one or more regulatory sequences operably linked to the polynucleotide sequence, where the regulatory sequence may include a suitable promoter sequence. The promoter sequence is usually operably linked to a sequence coding the amino acid sequence to be expressed. The promoter can be any nucleotide sequence that exhibits transcriptional activity in the selected host cell, including mutated, truncated and hybrid promoters, and can be obtained from a gene encoding an extracellular or intracellular polypeptide homologous or heterologous to the host cell. The regulatory sequence may further include a suitable transcription terminator sequence, a sequence recognized by the host cell to terminate the transcription. The terminator sequence is linked to the 3' end or terminus of the nucleotide sequence encoding the polypeptide, and any terminator that is functional in the host cell of choice may be used in the present disclosure.

Generally, a suitable vector may contain an origin of replication capable in at least one organism, a promoter sequence, a convenient restriction enzyme site and one or more selectable markers. For example, these promoters may include, but not limited to, the lac or trp promoter of *Escherichia coli* (*E. coli*); the lambda phage PL promoter; and eukaryotic promoters (including CMV immediate-early promoter, HSV thymidine kinase promoter, early and late SV40 promoters, methanol oxidase promoter of *Pichia pastoris*), and some other known promoters that are capable of controlling gene expression in prokaryotic cells or eukaryotic cells or viruses. Marker genes can be used to provide phenotypic characters for selection of transformed host cells. For example, marker genes may include, but not limited to, dihydrofolate reductase, neomycin resistance and green fluorescent protein (GFP) for eukaryotic cell culture, or tetracycline resistance or ampicillin resistance for *E*. *coli.* When the polynucleotide is expressed, the expression vector may further include an enhancer sequence. If an enhancer sequence is inserted into the vector, the transcription will be enhanced. Enhancer is a cis-acting factor of DNA, typically containing about 10 to 300 base pairs. Enhancer acts on a promoter to enhance gene transcription.

### Expression system

In a fifth aspect of the present disclosure, an antibody expression system is provided, which includes a construct provided in the fourth aspect or incorporates an exogenous polynucleotide provided in the third aspect of the present disclosure in the genome. Any cell suitable for the expression of an expression vector can be used as a host cell. For example, the host cell can be a prokaryotic cell, such as a bacterial cell; or a lower eukaryotic cell, such as a yeast cell; or a higher eukaryotic cell, such as a mammalian cell, specifically including, but not limited to, *Escherichia coli,* Streptomyces; bacterial cells of *Salmonella typhimurium;* or fungal cells such as yeast, and filamentous fungi; plant cells; insect cells derived from Drosophila S2 or Sf9; animal cells such as CHO, COS, HEK293 cells, or Bowes melanoma cells, or a combination thereof. Methods for constructing the expression system should be known to those skilled in the art, for example, including, but not limited to, microinjection, gene gun method, electroporation, virus-mediated transformation, electron bombardment, precipitation with calcium phosphate, or a combination thereof.

### Immunoconjugate

In a sixth aspect of the present disclosure, an immunoconjugate is provided, which includes the single-domain antibody provided in the first aspect of the present disclosure, or the fusion protein provided in the second aspect of the present disclosure. Generally the immunoconjugate may further includes a functional molecular linked to the single-domain antibody or fusion protein (including but not limited to covalent linkage, conjugation, attachment, and adsorption). The functional molecule may include, but not limited to, "detectable markers", cytotoxins, radioisotopes, biologically active proteins, molecules targeting tumor surface marker, tumor-suppressing molecules, molecules that target surface markers of immune cells, or a combination thereof.

Methods of preparing the immunoconjugate is known to those skilled in the art. For example, the single-domain antibody and/or fusion protein can be linked to the functional molecule directly or through a spacer of a suitable length, by chemical cross-linking or genetic engineering such as fusion expression, to obtain the immunoconjugate.

For therapeutic purposes, a therapeutic effector group such as a radioactive group may be suitable. The radioactive group are composed of a radioisotope or radionuclide (such as ³H, ¹⁴C, ¹⁵N, ³³P, ³⁵S, ⁹⁰Y, ⁹⁹Tc, ¹¹¹In, ¹²³I, ¹²⁵I, ¹³¹I, ²⁰¹Tl, ²¹³Bi), a toxin, or a cytotoxic group such as a cytostatic agent, or a group including the same.

The immunoconjugate may include the single-domain antibody or fusion protein of the present disclosure and a detectable marker. The detectable marker includes, but not limited to: a fluorescent marker, a developing marker, or a protein tag; such as an enzyme, a prosthetic group, a fluorescent material, a luminescent material, a bioluminescent material, a radioactive material, a positron-emitting metal and a non-radioactive paramagnetic metal ion. More than one markers may also be included. The labels used to label antibodies for detection and/or analysis and/or diagnostic purposes depend on the particular detection/analysis/diagnostic technique and/or method used, such as immunohistochemical staining of (tissue) samples, and flow cytometry, etc. Suitable labels for detection/analysis/diagnostic techniques and/or methods are well known to those skilled in the art.

The single-domain antibody or fusion protein of the present disclosure can be coupled with a labeling group (labeled polypeptide), and then used e.g. for diagnostic purposes. Suitable labeling groups may be selected from radioisotopes (such as those mentioned above) or groups containing radioisotopes orradionuclides, fluorophores (e.g. fluorescent proteins such as GFP and RFP, dyes, rhodamine, fluoresceins and derivatives thereof such as FITC and cyanine dyes), enzyme groups (e.g. horseradish peroxidase, alkaline phosphatase, and β-galactosidase), chemiluminescent groups, biotin groups, metal particles (e.g. gold particles), magnetic particles (for example, having cores containing magnetite (Fe₃O₄) and/or maghemite (Fe₂O₃)), and intended polypeptide groups, etc.

The immunoconjugate may include the single-domain antibody or fusion protein of the present disclosure and a molecule targeting a surface marker of immune cells. The molecule targeting a surface marker of immune cells can recognize immune cells, and carry the antibody of the present disclosure to reach immune cells. Also, the antibody of the present disclosure can target the immune cells to tumor cells, to exert the killing effect of the antibody of the present disclosure, and trigger the immune cells to specifically kill tumor cells.

The immunoconjugate may include the single-domain antibody or fusion protein of the present disclosure and at least one molecule targeting a tumor surface marker or a tumor-suppressing molecule. The tumor-suppressing molecule can be an anti-tumor cytokine, or an antitumor toxin. For example, the cytokine can be, but not limited to, IL-12, IL-15, IFN-beta, or TNF-alpha. The molecule targeting a tumor surface marker, for example, can take effects synergistically with the antibodies of the present disclosure, to more accurately target the tumor cells.

The immunoconjugate may further be a chimeric antigen receptor (CAR) expressed on immune cells. The term "immune cells" and "immune effector cells" are used interchangeably, and include T lymphocytes, NK cells or NKT cells, and preferably, NK cells and T lymphocytes. The chimeric antigen receptor generally includes an extracellular binding region, a transmembrane region and an intracellular signaling region connected in sequence. The extracellular binding region includes the single-domain antibody or fusion protein of the present disclosure. The design of the transmembrane region and the intracellular signaling region based on CAR technology is well known in the art. For example, the transmembrane region may be selected from CD8, CD28 and other molecules; and the intracellular signaling region is a CD3ζ chain of immunoreceptor tyrosine activation motif (ITAM), an FcεRlγ tyrosine activation motif or an intracellular signaling region of costimulatory signal molecules CD28, CD27, CD137, CD134, MyD88, CD40, and others. More specifically, For T lymphocytes, the first-generation CAR T lymphocytes only include ITAM in the intracellular signaling region, and the chimeric antigen receptor is linked in a pattern of scFv-TM-ITAM, to stimulate the anti-tumor cytotoxic effect. The second-generation CAR T lymphocytes has incorporated the intracellular signaling region of CD28 or CD137 (also known as 4-1BB), and the chimeric antigen receptor is linked in a pattern of scFv-TM-CD28 - ITAM or scFv-TM-/CD137-ITAM, where the co-stimulation of B7/CD28 or 4-1BBL/CD137 in the intracellular signaling region causes the continuous proliferation of T lymphocytes, increases the level of cytokines such as IL-2 and IFN-γ secreted by T lymphocytes, and improves the survival period and anti-tumor effect of CAR T in vivo. In the third-generation CAR T lymphocytes, the chimeric antigen receptor is linked in a pattern of scFv-TM-CD28-CD137-ITAM or scFv-TM-CD28-CD134-ITAM, further improving the survival period and anti-tumor effect of CAR T in vivo.

As described above, the chimeric antigen receptor prepared with the single-domain antibody or fusion protein of the present disclosure can include the following extracellular binding region, transmembrane region and intracellular signaling region linked in sequence: the single-domain antibody or fusion protein of the present disclosure, CD8 and CD3ζ; the single-domain antibody or fusion protein of the present disclosure, CD8, CD137 and CD3ζ; the single-domain antibody or fusion protein of the present disclosure, the transmembrane region (CD28a) of CD28 molecule, the intracellular signaling region (CD28b) of CD28 molecule, and CD3ζ; or, the single-domain antibody or fusion protein of the present disclosure, the transmembrane region of CD28 molecule, the intracellular signaling region of CD28 molecule, CD137 and CD3ζ.

Expression of the chimeric antigen receptor on the surface of immune effector cells, while exerting the killing effect of the antibody of the present disclosure, allows the immune effector cells to have a highly specific cytotoxic effect on tumor cells expressing PD-L1.

### Pharmaceutical composition

In a seventh aspect of the present disclosure, a pharmaceutical composition is provided, which includes an anti-PD-L1 single-domain antibody provided in the first aspect of the present disclosure, a fusion protein of an anti-PD-L1 single-domain antibody provided in the second aspect of the present disclosure, or an immunoconjugate provided in the sixth aspect of the present disclosure.

The pharmaceutical composition may also include various pharmaceutically acceptable carriers in the art. The pharmaceutically acceptable carrier is nontoxic to a recipient at the dose and concentration employed, and can specifically include, but not limited to, a buffering agent, such as an acetate, Tris, a phosphate, a citrate and other organic acids; an antioxidant, including ascorbic acid and methionine; a preservative (such as octadecyldimethylbenzylammonium chloride; hexamethonium chloride; benzalkonium chloride; benzethonium chloride; phenols, butanol or benzyl alcohol; alkyl paraben, such as methylparaben or propylparaben; catechol; resorcinol; cyclohexanol; 3-pentanol; and m-cresol); a protein, such as serum albumin, gelatin or immunoglobulin; a hydrophilic polymer, such as polyvinylpyrrolidone; an amino acid, such as glycine, glutamine, asparagine, histidine, arginine or lysine; a monosaccharide, a disaccharides and other carbohydrates, including glucose, mannose or dextrin; a chelating agent, such as EDTA; a tonicity modifier, such as trehalose and sodium chloride; a carbohydrate, such as sucrose, mannitol, trehalose or sorbitol; a surfactant, such as polysorbate; a salt-forming counter ion, such as sodium; a metal complex (such as a Zn-protein complex); and/or a nonionic surfactant, such as TWEE^{®}, PLURONICS^{®} or polyethylene glycol (PEG). The pharmaceutical formulation to be used for in-vivo therapeutic administration are generally sterile, and methods for sterilizing the pharmaceutical formulation are known to those skilled in the art, for example, filtration through a sterile filtration membrane. Those skilled in the art can also select a suitable pharmaceutically acceptable carrier according to the desired dosage form of the pharmaceutical composition, to prepare different dosage forms. For example, the pharmaceutical composition of the present disclosure can include, but not limited to tablets, injections, lyophilized formulation, and other dosage forms.

In the pharmaceutical composition, the fusion protein and immunoconjugate are usually in an effective amount, where the content of the active ingredient corresponding to the effective amount can be determined according to the subject to be treated and the specific mode of administration. For example, based on the total weight of the pharmaceutical composition, the content of the fusion protein and immunoconjugate can be in the range of about 0.01-99%, 0.1-70%, 1-30%, 0.01-0.05%, 0.05-0.1%, 0.1-0.3%, 0.3-0.5%, 0.5-1%, 1-3%, 3-5%, 5-10%, 10-20%, 20-30%, 30-50%, 50-70%, or 70-99%.

The fusion protein, immunoconjugate and pharmaceutical composition of the present disclosure can be administered as a single active ingredient, or administered in a combination therapy, i.e., in combination with other drugs. For example, the combination therapy includes a combination of the fusion protein, the immunoconjugate or the pharmaceutical composition with at least one other anti-tumor drug. For example, the combination therapy includes a combination of the fusion protein, the immunoconjugate or the pharmaceutical composition with an antibody targeting another tumor-specific antigen. The antibody targeting another tumor-specific antigen includes, but not limited to, anti-EGFR antibody, anti-VEGF antibody, anti-HER2 antibody, or anti-Claudin18A2 antibody, or a combination thereof. The inhibitor is preferably a monoclonal antibody.

### Detection kit

In an eighth aspect of the present disclosure, a detection kit is provided, which includes a single-domain antibody provided in the first aspect of the present disclosure, a fusion protein provided in the second aspect of the present disclosure, or an immunoconjugate provided in the sixth aspect of the present disclosure. The kit may further include, as required, a container, a control (negative or positive control), a buffer, and an auxiliary agent, etc., which can be selected by those skilled in the art according to the specific situation. An instruction for use may also be included in the kit, for the convenience of operation by those skilled in the art.

The present disclosure further provides a detection method for detecting PD-L1 protein by using the single-domain antibody, including, but not limited to, qualitative detection, quantitative detection and localization detection. Particularly, the detection method includes, but not limited to, immunofluorescence assay, immunohistochemistry and radioimmunoassay, etc.

A method for detecting the presence or absence of PD-L1 protein in a sample includes: contacting the sample with a single-domain antibody of the present disclosure; and observing whether an antibody complex is formed, where the formation of the antibody complex indicates the presence of PD-L1 protein in the sample. The sample may be a cell and/or tissue sample; where the sample is immobilized or dissolved in a medium, and the level of PD-L1 protein in the immobilized or dissolved sample is detected. In some embodiments, the detection object can be a cell-containing sample in a cell preservation solution. In some other embodiments, the single-domain antibody is also conjugated with a fluorescent dye, a chemical material, a polypeptide, an enzyme, an isotope, and a label that can be used for detection or can be detected by other reagents.

### Use

In a ninth aspect of the present disclosure, use of a single-domain antibody provided in the first aspect of the present disclosure, a fusion protein provided in the second aspect of the present disclosure, an immunoconjugate provided in the sixth aspect of the present disclosure, or a pharmaceutical composition provided in the eighth aspect of the present disclosure in the preparation of drugs for the diagnosis, treatment or prevention of diseases associated with cells expressing PD-L1.

The "therapeutically effective amount" of the fusion protein, the immunoconjugate, and the pharmaceutical composition provided in the present disclosure preferably causes a reduction in the severity of disease symptoms and increased frequency and duration of asymptomatic period of a disease, or prevents injury or disability due to illness or suffering. For example, for the treatment of PD-L1-related tumors (including, for example, melanoma), relative to untreated subjects, the "therapeutically effective amount" preferably inhibits the cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80%. The ability to inhibit tumor growth can be evaluated in an animal model system that predicts the efficacy against human tumors, or evaluated by detecting the ability to inhibit cell growth. Such inhibition can be determined in vitro by assays well known to those skilled in the art. The therapeutically effective amount of the fusion protein, the immunoconjugate, and the pharmaceutical compositions is often able to reduce the tumor size, or otherwise relieve the symptoms of a subject. Those skilled in the art can select an appropriate therapeutically effective dose according to the actual situation, for example, the size of the subject, the severity of the subject's symptoms, and the particular composition or route of administration chosen. A prescription for treatment (e.g., decision on dosage, etc.) may be determined by a physician commonly considering factors including, but not limited to, the disease being treated, status of the patient, delivery site, route of administration and other factors. A prophylactically effective amount refers to an amount effective for achieving the desired prophylactic effect at a dose and for a period of time required. Usually, but not necessarily, since a prophylactic dose is administered to a subject before the onset of a disease or at an early stage of the disease, the "prophylactically effective amount" is usually lower than the "therapeutically effective amount". Examples of diseases associated with PD-L1 expressing cells that can be diagnosed, treated and/or prevented in the present disclosure may include all PD-L1 expressing cancers and tumor entities, specifically including, but not limited to, lung cancer, melanoma, gastric cancer, ovarian cancer, colon cancer, liver cancer, kidney cancer, bladder cancer, breast cancer, classic Hodgkin lymphoma, hematological malignancies, head and neck cancer and nasopharyngeal cancer, which may be early, intermediate, or advanced, such as, metastatic cancers.

The implementations of the present disclosure will be described below by way of specific examples, and other advantages and effects of the present disclosure are readily comprehensible to those skilled in the art from the disclosure of the present disclosure. The present disclosure can also be implemented or applied through other different specific embodiments, and various modifications or changes can be made to the details in the specification based on different viewpoints and applications, without departing from the spirit of the present disclosure.

Before further describing specific embodiments of the present disclosure, it should be understood that the protection scope of the present disclosure is not limited to the following specific embodiments. It should also be understood that the terms used in the examples of the present disclosure are for the purpose of describing specific embodiments, instead of limiting the scope of protection of the present disclosure.

When a numerical range is given in the example, it should be understood that both endpoints of each numerical range and any value between the two endpoints can be used, unless otherwise specified in the present disclosure. All technical and scientific terms used in the present disclosure have the same meaning as commonly understood by those skilled in the art, unless otherwise defined. In addition to the specific method, apparatus, and material used in the examples, according to the understanding of the prior art by those skilled in the art and the description of the present disclosure, any method, apparatus, apparatus, and material in the prior art that are the same as or similar to the method, apparatus, and material described in the examples of the present disclosure can be used to implement the present disclosure.

Unless otherwise stated, the experimental methods, detection methods, and preparation methods disclosed in the present disclosure are all conventional techniques used in molecular biology, biochemistry, chromatin structure and analysis, analytical chemistry, cell culture, and recombinant DNA technology in the art, as well as conventional techniques in related art. These techniques are well described in the existing literatures.

### Example 1 Construction of anti-PDL1 single-domain antibody library

1 mg PDL1-HSA fusion protein (SEQ ID NO: 49) composed of PDL1 extracellular domain and human serum albumin was mixed with 1 ml of Freund's complete adjuvant (sigma), and emulsified. Healthy alpacas (*Vicugnapacos*) were immunized and then further immunized after 21 days for a total of three immunizations, to stimulate B cells to express the antigen-specific single-domain antibody. One week after the third-immunization, 30 ml of alpaca blood was collected with a vacuum blood collection tube, lymphocytes were separated using a lymphocyte separation medium (Tianjin HaoyangHuake Biotechnology Co., Ltd.), and total RNA was extracted by Trizol method. 3 µg of total RNA was reverse transcribed into cDNA using a reverse transcription kit (Invitrogen) according to the manufacturer's instructions, and VHH was amplified by nested PCR. The target VHH nucleic acid fragments were recovered, digested with restriction endonuclease Sfil (NEB), inserted into the digested phage display vector pcomb3xss (Addgene plasmid # 63890; RRID:Addgene_63890), and ligated by T4 ligase (Takara). The ligation product was transformed into electrocompetent cells ER2738, to construct an anti-PDL1 single-domain antibody library. The cells were plated by gradient dilution, and the size of the library capacity was determined to be 1.25 × 10⁸. Further, 24 clones were randomly selected for PCR detection. The results show that the insertion rate of the constructed library was 100%.

### Example 2 Screening and identification of anti-PDL1single-domain antibody

### 2.1. Screening of Anti-PDL1single-domain antibody

The constructed anti-PDL1 single-domain antibody library was packaged with helper phage M13KO7 (NEB), and the titer of the recombinant phages in the display library was 2.88 × 10¹³ PFU/ml. PDL1-HSA was diluted with a coating buffer of 100 mM NaHCO₃( pH=8.2) to 2 µg/ml and added in 100 µL/well to a microtiter plate, and allowed to stand at 4°C overnight. On the following day, 200 µL of 3% BSA was added to the plate and incubated at 37°C for 1 hr. 100 µL of recombinant phage (about 2.88 × 10¹¹PFU/well, obtained from the library constructed in Example 1) was added, and incubated for 1 hr at room temperature. Then the plate was washed 5 times with PBST (containing 0.1% Tween 20), to remove non-specifically binding phages. After washing, the plate was incubated for 10 min with 1 ml of 0.1 M gly-HCI and 1 mg/ml BSA (pH2.2) buffer and eluted off, and then neutralized with 1 M Tris-HCl pH 8.0. The phage titer of the above round of panning was 5.25 × 10⁶ PFU/ml. The phage eluted from the above round of panning was amplified, and the titer was 1.3 × 10¹³ PFU/ml. The fusion protein PDL1-Fc (SEQ ID NO: 50) composed of PDL1 extracellular domain sequence and human IgG1 Fc was used as a coating protein, 3% ovalbumin (OVA) was used for blocking, and the second round of panning was carried out following the same screening process. The titer of phage after the second round of panning was 2.54 × 10⁸ PFU/ml.

### 2.2. Double screening by Enzyme-linked immunosorbent assay (ELISA) and TEPITOPE

380 single clones were picked from the phage titer determination plate eluted after the second round of panning, cultured in 96-well plates, infected and packaged with M13KO7 helper phage, to obtain the accumulated recombinant phages in the supernatant. The plate was coated with PDL1-HSA in 200 ng/well, and blocked with 3% BSA for 1 hr at room temperature. ELISA was carried out in the presence of various concentrations of PD1-FC (SEQ ID NO: 51), a binding curve was plotted, and the EC90 was calculated to be 2.4 µg/mL. The monoclonal recombinant phage-containing supernatant were mixed in equal proportion with 4 µg/mL PD1-FC in a 96-well plate, and incubated in the 96-well plate coated with PDL1-HSA in an amount of 100 µl/well (where the well with only PD1-FC at a final concentration of 2 µg/mL was used as a control) at 37°C for 1 hr. After washing three times with PBST, 100 µl of 1:20000 diluted HRP- Goat anti-Human IgG Fc(Novex) was added to each well, and the plate was incubated at 37°C for 1 hr. After washing 3 times with PBS, a TMB developing solution (Beijing ComWin Biotech Co.,Ltd) was added, and the plate was incubated for 5 min at room temperature for color development. 1 M sulfuric acid was added to stop the reaction, and the OD value was read at 450 nm. The PD1-Fc in the control well showed obvious color development (with an OD450nm value of 3.375), and wells with phage competition-positive clones showed weak or almost no color development (with an OD450nm value of lower than 0.3). Competition-positive clones were sequenced, and the repeated clones were removed. The immunogenicity of the obtained high-affinity positive clones was further analyzed, and the scores of alleles DRB1*03:01, DRB1*07:01, DRB1*15:01, DRB3*01:01, DRB3*02:02, DRB4*01:01 and DRB5*01:01 which are closely related to human immunogenicity were calculated using the prediction tool TEPITOPE based on the QAM method (Sturniolo T et al., Nat. Biotechnol. 17:555-561). Furthermore, sequences with a total CDR3 TEPITOPE score above 4 were excluded to obtain highly humanized anti-PD-L1 single-domain antibodies after screening. Most of the total CDR3 TEPITOPE scores of the anti-PD-L1 single-domain antibodies obtained in the present disclosure are <-2.0, which are significantly lower than that of similar anti-PD-L1 single-domain antibodies, suggesting that the anti-PD-L1 single-domain antibodies of the present disclosure have a lower potential risk of immunogenicity.

After several rounds of screening, the present inventors finally picked out 6 positive anti-PDL1 single-domain antibody sequences which showed strong competition and low TEPITOPE scores. Their full-length sequences are shown in Table 1a, where the CDR regions are underlined.

**Table 1a**

| | Full-length sequence | SEQ ID NO: |
|---|---|---|
| 1A7 | QLQLVESGGDLVQPGGSLRLSCAASGSIFSANLIDWYRQPPG KQRELVAVISSRGITSYADSVKGRFTISRDNAKNTVYLQMNSLK PEDTAVYYCHLFHTDGLGYWGQGTQVTVSS | 33 |
| 1D1 | QLQLVESGGGLVQAGGSLRLSCAASGGTFITYAMGWFRQAP GKDREFVAGISWSGSSTYYTDSVKGRFTISRDNAKNTVYLQM NSLKPGDTAVYYCAAKISGATREHLMTSYDYWGQGTQVTVS S | 34 |
| 1D7 | QVQLVESGGGLVQPGGSLRLSCAASGRAFLTYAMGWFRQAP GKEREFVAAISWSGSSTYYADSVKGRFTISRDNAKNMVYLQM NSLKPEDTAVYYCAAKVSGATRDHLMTTYDYWGQGTQVTVS S | 35 |
| 1G10 | QVQLVESGGGLVQAGGSLRLSCAASGRPFITYAMGWFRQAP GKEREFVAAINWSGSITAYADSVKGRFTISRDNAKNAAYLQMN SLKPDDTAVYYCASKRTAVAPRALNDYDFWGQGTQVTVSS | 36 |
| 5A8 | QLQLVESGGGLVQPGGSLRLSCAASGGTFITYAMGWFRQAP GKDREFVAGISWSGSSTYYTDSVKGRFTISRDNAQNTVYLQM NSRKPGDTAVYYCAAKISGATREHLRTSYDYGGQGTQVTVSS | 37 |
| 6F1 | QLQLVESGGGLVQPGGSLRLSCAASGRTFSTHSMHWFRQAP EKERVFVAAINWNGDSTYYADSVKGRFTISRDNSKNTVDLQM NNLKPEDTAVYVCAARPGAPSTIVAMLDYWGQGTQVTVSS | 38 |

The framework region (FR) and complementarity determining region (CDR region) of each antibody are shown in Table 1b.

**Table1b**

| | FR1(SE Q ID NO:) | CDR1(SE Q ID NO:) | FR2(SE Q ID NO:) | CDR2(SE Q ID NO:) | FR3(SE Q ID NO:) | CDR3(SE Q ID NO:) | FR4(SE Q ID NO:) |
|---|---|---|---|---|---|---|---|
| 1A7 | 1 | 6 | 11 | 15 | 19 | 25 | 31 |
| 1D1 | 2 | 7 | 12 | 16 | 20 | 26 | 31 |
| 1D7 | 3 | 8 | 13 | 16 | 21 | 27 | 31 |
| 1G1 0 | 4 | 9 | 13 | 17 | 22 | 28 | 31 |
| 5A8 | 5 | 7 | 12 | 16 | 23 | 29 | 32 |
| 6F1 | 5 | 10 | 14 | 18 | 24 | 30 | 31 |

The TEPITOPE score of CDR3 was shown in Table 1c.

**Table 1c**

| Clone No. | CDR3 SEQ ID NO. | TEPITOPE score (Threshold: 3%) | | | | | | | |
|---|---|---|---|---|---|---|---|---|---|
| | | DRB1* 03:01 | DRB1* 07:01 | DRB1* 15:01 | DRB3* 01:01 | DRB3* 02:02 | DRB4* 01:01 | DRB5* 01:01 | Total score |
| 1A7 | 25 | -0.3165 | 4.0793 | 2.0981 | -1.5304 | -1.7309 | -1.2642 | 1.8849 | 3.5368 |
| 1D1 | 26 | 0.3353 | 2.071 | 0.6483 | -0.3405 | -1.9074 | -0.8487 | -2.0181 | -2.0601 |
| 1D7 | 27 | -0.1001 | -0.1069 | 0.6146 | -0.8096 | -2.6263 | -1.3442 | -2.6174 | -6.9899 |
| 1G10 | 28 | -0.4723 | -0.222 | 0.4577 | -1.4754 | -2.6443 | -1.0317 | -1.6145 | -7.0025 |
| 5A8 | 29 | 0.3353 | 2.071 | 0.6483 | -0.3405 | -1.9074 | -0.8487 | -2.0181 | -2.0601 |
| 6F1 | 30 | -8.9 | -10.2 | -8 | -10 | -12.6 | -10 | -10.5 | -70.2 |
| PDL1-56 dAb | 66 | 1.99 | 3.873 | 0.7435 | 1.5116 | 1.2524 | 2.5062 | 1.4777 | 13.354 4 |
| Innoven tNb | 67 | 2.5389 | 3.5707 | 3.0883 | 1.9171 | 1.0934 | 1.8357 | 3.5789 | 17.623 |
| Nb 43 | 68 | 1.3009 | 2.886 | 2.3973 | -0.1293 | -0.466 | -0.4211 | 3.163 | 8.7308 |

Note: PDL1-56 dAb is derived from WO2017/020801A1, InnoventNb is derived from CN107686520A, Nb 43 is derived from WO2018/233574A1. The TEPITOPE of amino acids 2-26 in CDR3 of InnoventNb is predicted.

### Example 3 Preliminary evaluation and identification of anti-PDL1 single-domain antibody

### 3.1. Expression of single-domain antibodies in E. coli and purification

Using the screened specific positive sequence as a template, PCR amplification was performed using the high-fidelity enzyme GVP8 (General Biosystems (Anhui) Co., Ltd.). A histidine tag coding sequence was introduced at the 3' end of the sequence. The PCR product was subjected to electrophoresis and extracted to recover a band of about 600 bp. The recovered PCR product was recombinantly ligated to the pET32a+ vector (Novagen) digested with endonuclease Ndel (NEB) and EcoRI (NEB) using a recombination kit (Novoprotein Scientific Co., Ltd.), to construct an *E. coli* expression plasmid. The plasmid was transformed into competent *E. coli* Top10F' cells which were plated onto an ampicillin-coated plate, followed by incubation overnight at 37°C. The clones on the ampicillin-coated plate were picked respectively, and the plasmid was extracted and sequenced, to confirm the correct insertion of the sequence into the pET32a+ vector. The *E. coli* expression plasmid confirmed by sequencing was transformed into *E. coli* expression host Rosetta (DE3), to construct an *E. coli* expression strain. The recombinant clones on an ampicillin plate was picked up, incubated, and induced with 1 mM IPTG to express overnight at 30°C. The cell suspension induced to express overnight was ultrasonicated, and subjected to centrifugation at 12,000g and 4°C for 10 min. The supernatant was collected, and purified by a Ni column (BestChrom Biotechnology Co., Ltd.). The protein showed purity above 90%.

### 3.2. Specific binding of anti-PDL1 single-domain antibody to human PD-L1

Human PDL1-HSA or HSA (purchased from Sigma) was coated onto a plate in an amount of 200 ng/well at 4°C, and blocked with 5% skimmed milk powder for 1 hr at room temperature. The purified anti-PDL1 single-domain antibody with a histidine-tag was diluted to 1 µg/mL, added to the plate in an amount of 100 µL/well, and incubated at 37°C for 1 hr. After washing three times with PBST, 100 µl/well of 1:5000 diluted mouse anti-his tag antibody (R&D Systems Inc.) was added, and the plate was incubated at room temperature for 1 hr. After washing with PBST, 100 µl/well of 1:10000 diluted HRP-Goat anti mouse IgG antibody (Thermo Scientific) was added, and the plate was incubated at room temperature for 1 hr. After washing with PBST, a TMB developing solution (Beijing ComWin Biotech Co.,Ltd) was added for color development, and the OD value was read at 450 nm.

The test results are shown in Table 2. The results show that the six clones screened above specifically bind to human PDL1.

**Table2**

| Clone No. | OD450nm (coated with PDL1-HSA) | OD450nm (coated with HSA) |
|---|---|---|
| 1A7 | 2.051 | 0.082 |
| 1D1 | 2.013 | 0.074 |
| 1D7 | 2.152 | 0.076 |
| 1G10 | 2.083 | 0.073 |
| 5A8 | 2.024 | 0.064 |
| 6F1 | 2.053 | 0.081 |

### 3.3. Binding of anti-PDL1 single-domain antibody to mouse PD-L1

Mouse mPDL1-HSA fusion protein (SEQ ID NO: 52) was coated onto a plate overnight in an amount of 200 ng/ well at 4°C, and blocked with 5% skimmed milk powder for 1 hr at room temperature. The purified histidine-tagged anti-PDL1single-domain antibody was diluted to 1 µg/mL, then added to the plate in an amount of 100 µL/well, and incubated at 37°C for 1 hr. After washing three times with PBST, 100 µl/well of 1:5000 diluted mouse anti his tag antibody (R&D Systems Inc) was added, and the plate was incubated at room temperature for 1 hr. After washing with PBST, 100 µl/well of 1:10000 diluted HRP-Goat anti mouse IgG antibody (Thermo Scientific) was added, and the plate was incubated at room temperature for 1 hr. After washing with PBST, TMB was added for color development, and the OD value was detected at 450 nm.

The results showed that the OD value corresponding to the anti-PDL1 single-domain antibody was not significantly different from that of the blank control group without the anti-PDL1single-domain antibody, indicating that none of the six clones screened bind to mouse PDL1.

### 3.4. Competitive ELISA detection of Anti-PDL1 single-domain antibody protein

PDL1-HSA was coated onto a plate overnight in an amount of 200 ng/well at 4°C, and blocked with 5% skimmed milk powder for 1 hr at room temperature. The purified histidine tagged anti-PDL1 single-domain antibody was diluted in gradient and then mixed with an equal volume of 4.8 µg/mL PD1-Fc, respectively. 100 µl was added per well and incubated at 37°C for 1 hr. After washing three times with PBST, 100 µl of 1:20000 diluted HRP- Goat anti-Human IgG Fc(Novex) was added to each well, and incubated at room temperature for 1 hr. After washing three times with PBS, TMB substrate was added, and the plated was incubated at 37°C. After incubation for 5 min to develop color, 1 M sulfuric acid was added to stop the reaction. The OD value was read at 450 nm, and the half maximal inhibitory concentration (IC50 value) was calculated. A smaller IC₅₀ value indicates a higher specificity of the antibody.

The results are shown in Table 3, showing that the single-domain antibodies screened in the present disclosure have a good binding specificity.

**Table 3**

| Clone No. | IC50 (ng/ml) |
|---|---|
| 1A7 | 50.5 |
| 1D1 | 45.2 |
| 1D7 | 55.1 |
| 1G10 | 62.8 |
| 5A8 | 43.6 |
| 6F1 | 52.7 |

### Example 4. Humanization of anti-PDL1 single-domain antibodies

The humanization was carried out by the VHH humanization universal framework transplantation method established by Vincke C et al. (Vincke C, Loris R, Saerens D, Martinez-Rodriguez S, Muyldermans S, Conrath K. J Biol Chem. 2009; 284(5): 3273-3284). The corresponding CDR region in the universal humanized VHH framework h-NbBcII10FGLA (PDB number: 3EAK) designed according to sequence homology was replaced by the CDR region of the anti-PDL1 single-domain antibody, and individual amino acids in the FR2 region were further humanized according to the sequence of the humanized antibody DP-47. Multiple humanized variants were obtained for each anti-PDL1 single-domain antibody. On basis of introducing as many human-derived amino acids as possible, a humanization scheme with high solubility and less aggregation was used. The humanized single-domain antibody was expressed and purified according to Example 3.1.

Preferably, the humanized VHH has an amino acid sequence of SEQ ID NO: 39-44, as shown in Table 4a, where the CDR regions are underlined.

**Table 4a**

| | Full-length sequence | SEQ ID NO: |
|---|---|---|
| hu1A7V 3 | QVQLVESGGGLVQPGGSLRLSCAASGSIFSANLIDWYRQAP GKGLELVAVISSRGITSYADSVKGRFTISRDNSKNTLYLQMNS LRAEDTAVYYCHLFHTDGLGYWGQGTLVTVSS | 39 |
| hu1D1V 3 | QVQLVESGGGLVQPGGSLRLSCAASGGTFITYAMGWFRQA PGKGLEFVAGISWSGSSTYYADSVKGRFTISRDNSKNTLYLQ MNSLRAEDTAVYYCAAKISGATREHLMTSYDYWGQGTLVTV SS | 40 |
| hu1D7V 3 | QVQLVESGGGLVQPGGSLRLSCAASGRAFLTYAMGWFRQA PGKGLEFVAAISWSGSSTYYADSVKGRFTISRDNSKNTLYLQ MNSLRAEDTAVYYCAAKVSGATRDHLMTTYDYWGQGTLVT VSS | 41 |
| hu1G10 V3 | QVQLVESGGGLVQPGGSLRLSCAASGRPFITYAMGWFRQA PGKGLEFVAAINWSGSITAYADSVKGRFTISRDNSKNTLYLQ MNSLRAEDTAVYYCASKRTAVAPRALNDYDFWGQGTLVTV SS | 42 |
| hu5A8V 3 | QVQLVESGGGLVQPGGSLRLSCAASGGTFITYAMGWFRQA PGKGLEFVAGISWSGSSTYYADSVKGRFTISRDNSKNTLYLQ MNSLRAEDTAVYYCAAKISGATREHLRTSYDYWGQGTLVTV SS | 43 |
| hu6F1V 3 | QVQLVESGGGLVQPGGSLRLSCAASGRTFSTHSMGWFRQA PGKGLEFVAAINWNGDSTYYADSVKGRFTISRDNSKNTLYLQ MNSLRAEDTAVYYCAARPGAPSTIVAMLDYWGQGTLVTVSS | 44 |

The framework region (FR) and complementarity determining region (CDR region) of each humanized variant are shown in Table 4b.

**Table 4b**

| | FR1(SE Q ID NO:) | CDR1(SE Q ID NO:) | FR2(SE Q ID NO:) | CDR2(SE Q ID NO:) | FR3(SE Q ID NO:) | CDR3(SE Q ID NO:) | FR4(SE Q ID NO:) |
|---|---|---|---|---|---|---|---|
| hu1A7V3 | 3 | 6 | 59 | 15 | 62 | 25 | 65 |
| hu1D1V3 | 3 | 7 | 60 | 16 | 63 | 26 | 65 |
| hu1D7V3 | 3 | 8 | 61 | 16 | 63 | 27 | 65 |
| hu1G10 V3 | 3 | 9 | 61 | 17 | 64 | 28 | 65 |
| hu5A8V3 | 3 | 7 | 60 | 16 | 63 | 29 | 65 |
| hu6F1V3 | 3 | 10 | 61 | 18 | 63 | 30 | 65 |

The degree of humanization of each humanized single-domain antibody (by comparison of sequence identity to the closest human gene and allele) was analyzed by IMGT/3Dstructure-DB (http://www.imgt.org), as described in Use of IMGT® Databases and Tools for Antibody Engineering and Humanization, Marie-PauleLefranc et al., Methods Mol Biol, 907:3-37, 2012. The results are shown in Table 4c.

**Table 4c**

| | Closest human gene and allele | Sequence identity (%) |
|---|---|---|
| hu1A7V3 | IGHV3-66*01, IGHJ4*01 | 84.2 |
| hu1D1V3 | IGHV3-23*04, IGHJ4*01 | 88.7 |
| hu1D7V3 | IGHV3-23*04, IGHJ4*01 | 88.7 |
| hu1G10V3 | IGHV3-23*04, IGHJ4*01 | 85.6 |
| hu5A8V3 | IGHV3-23*04, IGHJ4*01 | 88.7 |
| hu6F1V3 | IGHV3-64*04, IGHJ4*01 | 87.6 |
| hu56V1 | IGHV3-64*04, IGHJ1*01 | 78.4 |
| hu56V2 | IGHV3-64*04, IGHJ1*01 | 81.4 |
| InnoventNb | IGHV3-66*01, IGHJ5*02 | 81.2 |
| HualanNb 1 | IGHV3-NL1*01, IGHJ6*01 | 72.2 |
| HualanNb 2 | IGHV3-23*01, IGHJ4*01 | 78.4 |
| Bevacizumab VH | IGHV3-30*02, IGHJ4*01 | 76.8 |
| Adalimumab VH | IGHV3-9*01, IGHJ4*01 | 93.9 |

Note: hu56V1 and hu56V2 are respectively derived from SEQ ID NO:33 and SEQ ID NO:34 of WO2017/020801A1, Innovent Nb is derived from SEQ ID NO:14 of CN107686520A, and Hualan Nb 1 and Hualan Nb 2 are respectively derived from SEQ ID NO: 8 and SEQ ID NO: 14 of WO2018/233574A1. Bevacizumab and Adalimumab are marketed monoclonal antibodies.

### Example 5. Preparationof anti-PDL1-Fc fusion protein with mammalian cells

### 5.1. Expression and purification of anti-PDL1 single-domain antibodies and Fc fusion protein (Anti-PDL1-Fc)

The amino acid sequences of the screened specific positive clones (SEQ ID NO: 33-38) and the humanized counterparts (SEQ ID NO: 39-44) were respectively converted into a base sequence according to the codon preference in CHO cells, and full-length DNAs were obtained by gene synthesis (Nanjing GenScript Biotechnology Co., Ltd.). Using each DNA as a template, PCR amplification was performed using the high-fidelity enzyme GVP8 (General Biosystems (Anhui) Co., Ltd.). The PCR product was subjected to electrophoresis and extracted to recover a band of about 600 bp. The recovered PCR product was recombinantly ligated to the pCDNA3.1 vector containing a signal peptide and human IgG1 Fc sequence (SEQ ID NO: 45), to construct a cell expression plasmid in which anti-PDL1 single-domain antibody is fused with human IgG1 Fc. The expression plasmid in which anti-PDL1 single-domain antibody was fused with human IgG1 Fc was extracted with an endotoxin-free plasmid max kit (Biomiga). The plasmid was mixed well with the transfection reagent PEI (Polysciences, Inc.) at a ratio of 1:3, and allowed to stand for 30 min, and then added to HEK293F cells. After 7 days of culture in a shaker incubator at 37°C and 5%CO₂, the cell suspension was centrifuged, and the supernatant was collected. The supernatant was adjusted to pH 7.0, loaded onto a Protein A affinity column (Bestchrom Biotechnology Co., Ltd), and then eluted with 100% 0.1 M Gly-HCl (pH3.0). The eluate was pre-added with 10% 1M Tris-HCl (pH 8.5), diluted to a conductivity of 4ms/cm with 100% eluant, adjusted to pH 5.5, and centrifuged (8000 rpm, 4°C, 10 min). The supernatant was adjusted to pH 5.0 and loaded onto a DSP column (Bestchrom Biotechnology Co., Ltd), and eluted in linear gradients with 0-60% eluant (20mM NaAc, 0.5M NaCl, pH5.0), at a flow rate of 2 ml/min for 15 min. The purity was determined by SEC-HPLC-UV analysis under the following conditions: detector: Agilent 1100 LC; detection wavelength: 214 nm; mobile phase: 150 mM pH7.0 PB + 5% isopropanol; chromatographic column: Superdex 200 Increase 5/150 GL; running time: 15 min; column temperature: 25°C. The purity is greater than 95%.

### Example 6. Identification of in-vitro functions of anti-PDL1-Fc fusion protein

### 6.1. Affinity characterization of anti-PDL1-Fc fusion protein to PD-L1

PDL1-HSA was coated onto a plate overnight in 200 ng/well at 4°C, and blocked with 5% skimmed milk powder for 1 hr at room temperature. The anti-PDL1-Fc fusion protein was diluted in gradient with 1% BSA, and incubated at 37°Cfor 1 hr. After washing three times with PBST, 100 µl of a 1:20000 dilution of HRP- Goat anti-Human IgG Fc(Novex) was added to each well, and incubated at room temperature for 1 hr. After washing three times with PBST, TMB substrate was added, and the plate was incubated at 37°C. After incubation for 5 min to develop color, 1 M sulfuric acid was added to stop the reaction, and the OD value was read at 450 nm. The results are shown in Table 5.

**Table 5**

| Sample name | EC50(nM) |
|---|---|
| Anti-PDL1-1A7-FC | 0.07 |
| Anti-PDL1-1D1-FC | 0.07 |
| Anti-PDL1-1D7-FC | 0.07 |
| Anti-PDL1-1G10-FC | 0.08 |
| Anti-PDL1-5A8-FC | 0.11 |
| Anti-PDL1-6F1-FC | 0.09 |
| Anti-PDL1-1A7V3-Fc | 0.09 |
| Anti-PDL1-1D1V3-Fc | 0.10 |
| Anti-PDL1-1D7V3-Fc | 0.08 |
| Anti-PDL1-1G10V3-Fc | 0.10 |
| Anti-PDL1-5A8V3-Fc | 0.08 |
| Anti-PDL1-6F1V3-Fc | 0.09 |

### 6.2. Identification of blocking effect of anti-PD-L1 single-domain antibody/Fc fusion protein on PDL1-PD1 interaction (by competitive ELISA)

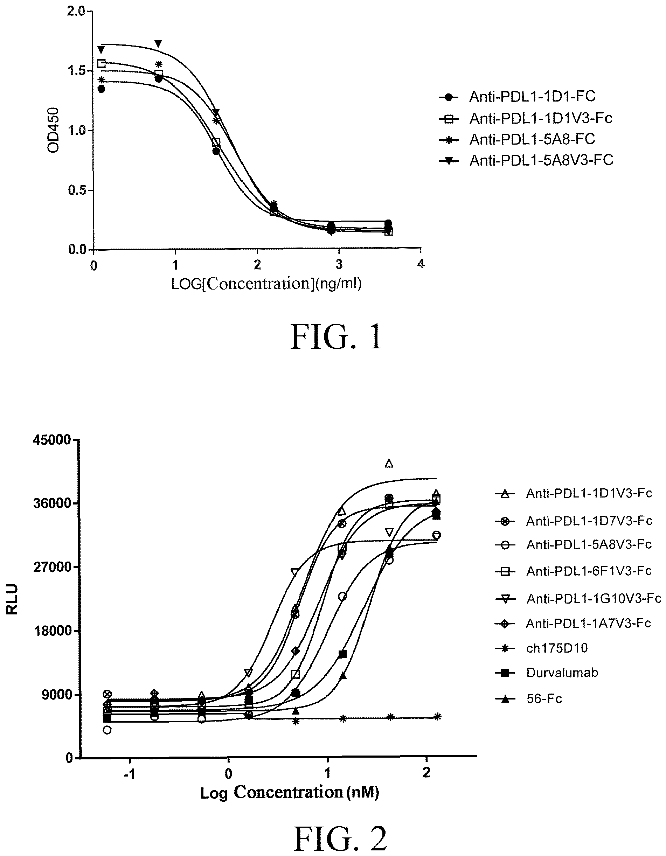

PDL1-HSA was coated onto a plate overnight in an amount of 200 ng/well at 4°C, and blocked with 5% skimmed milk powder for 1 hr at room temperature. The anti-PDL1-Fc fusion protein was diluted 5-fold in gradient with 1% BSA from an initial concentration of 4 µg/mL, and mixed with an equal volume of 4 µg/mL bio-PD1-F_{C}(biotin-labeled), respectively. 100 µL of the mixture was added to a 96-well plate and incubated at 37°C for 1 hr. After washing three times with PBST, 100 µL of a 1:50,000 dilution of HRP-Streptavidin (Jackson ImmunoResearch Inc.) was added to each well, and the plate was incubated at room temperature for 1 hr. After washing three times with PBST, TMB substrate was added, and the plate was incubated at 37°C. After color development for 5 min, 1 M sulfuric acid was added to stop the reaction. The OD value was read at 450 nm, and the results are shown in Table 6 and Fig. 1.

**Table 6**

| Sample name | IC50(ng/ml) |
|---|---|
| Anti-PDL 1-1A7-FC | 43.52 |
| Anti-PDL1-1D1-FC | 33.50 |
| Anti-PDL1-1D7-FC | 43.31 |
| Anti-PDL 1-1G10-FC | 41.22 |
| Anti-PDL 1-5A8-FC | 46.79 |
| Anti-PDL 1-6F1-FC | 39.18 |
| Anti-PDL 1-1A7V3-Fc | 33.50 |
| Anti-PDL1-1D1V3-Fc | 34.60 |
| Anti-PDL 1-1D7V3-Fc | 37.74 |
| Anti-PDL 1-1G10V3-Fc | 38.65 |
| Anti-PDL1-5A8V3-Fc | 33.50 |
| Anti-PDL1-6F1V3-Fc | 39.62 |

The above results show that the single-domain antibodies of the present disclosure have a very significant blocking effect on PDL1-PD1 interaction.

### Example 7. Identification of functional activity of anti-PDL1-Fc fusion protein in human T lymphocyte lineage

### 7.1. Construction of assay cell strains

CD5L-OKT3scFv-CD14 (GenBank: ADN42857.1) was synthesized, digested with HindIII-EcoRI (Takara), and inserted into the vector pCDNA3.1, to construct pCDNA3.1-antiCD3TM. Human PD-L1 was used as a template, high-fidelity amplification was performed to obtain a PDL1 fragment, which was then recombinantly inserted into pCDNA3.1-antiCD3TM, to construct pCDNA3.1-antiCD3TM-PDL1. CHO cells (Thermo) were transfected and screened against G418 for 10-14 days to produce the stable cell line CHO-antiCD3TM-PDL1.

The obtained fragment was amplified with human PD1 as a template, and then recombinantly ligated to the vector PB513B1-dual-puro (YouBio) digested with HindIII-BamHI (Takara), to construct the plasmid pB-PD1. pGL4.30 (YouBio) was used as a template, the obtained fragment was subjected to high-fidelity amplification, and then recombinantly ligated to the vector pB-PD1 digested with Sfil-Xbal (Takara), to construct the plasmid pB-NFAT-Luc2p-PD1. After the plasmid was successfully constructed, the plasmid was extracted with an endotoxin-free plasmid maxiprep extraction kit (Biomiga), with which jurkat cells (Stem Cell Bank, Chinese Academy of Sciences) were transfected. Following the method described in Patent No. CN 107022571A, jurkat cells were allowed to be in relatively adherent state by treatment with 0.1 mg/ml poly-D-lysine, and then the jurkat cells were transfected according to instructions for transfection in a lipofection kit (Lipofectamine 3000; invitrogen). On the third day, the cells were screened under 10% FBS-containing RPMI1640 medium (Thermo) containing 2.5 µg/ml puromycin. After that, the medium was supplemented at regular intervals. After the cell viability was restored, the content of puromycin was gradually increased to 4 µg/ml. The Jurkat-NFAT-Luc2p-PD1 cell line was finally obtained.

### 7.2. Characterization of blocking capacity of anti-PDL1-F_{C} fusion proteins in PDL1/ PD1 pathway

CHO-CD3TM-PD-L1 and Jurkat-NFAT-Luc2p-PD-1 cells were counted, and the cell density thereof was adjusted to be 4 × 10⁶/ml. Then 25 µl was added to each well of a 96-well plate. In this experiment, the anti-PDL1-Fc fusion protein, positive controls Durvalumab(AstraZeneca) and 56-Fc (SEQID NO:42 from WO2017/020801A1), and negative control ch-175D10 (from US9751934B2) were respectively diluted in gradient with 1% BSA, and 50 µl were added to the cells, to give a final concentration of 130, 43.4, 14.5, 4.82, 1.61, 0.536, 0.179, and 0.0595 nM; and coincubated at 37°C and 5%CO₂ for 6 hrs. 10 µl of luciferase substrate was added to each well, and shaken on a shaker for 2 min, and then the luminescence was read. The operation was carried out according to the instruction of the kit (luciferase detection kit; Lot: 0000339727; Promega).

The results in Fig. 2 shows that after addition of the anti-PDL1 antibody, the binding of PD-1/PD-L1 between effector cells and target cells was blocked specifically in a dose dependent manner. ch-175D10 (targeting Claudin18.2) did not block the binding of PD-L1 and PD1.

It can be seen from Fig. 2 that the ability of the anti-PDL1 antibody optimized in the present disclosure to block the binding of PD-1/PD-L1 on the surface of the effector and target cells is better than that of the positive controls Durvalumab and 56-Fc.

### Example 8. Characterization of activation of PBMC by anti-PDL1-Fc fusion protein

PBMCs were isolated from healthy human peripheral blood using a lymphocyte separation medium (TianjinHaoyangHuake Biotechnology Co., Ltd.). The recombinant anti-CD3 monoclonal antibody (Novoprotein Scientific Co., Ltd., Article No. GMP-A018) at a final concentration of 5 µg/ml was coated onto a plate overnight at 4°C in an amount of 50 µl/well. The cells were diluted to 2 × 10⁶/ml with 2 µg/ml recombinant CD28 monoclonal antibody (Novoprotein Scientific Co., Ltd., Article No. GMP-A063). The coated plate was removed of the supernatant, and washed twice with 200 µl/well of PBS. 200 µl of cells was added to each well. After 24 hrs of cell activation, 5 nM Durvalumab(AstraZeneca) and 5nM Anti-PDL1-Fc fusion protein were added for stimulation. After 3 days of stimulation, the cell suspension was centrifuged at 1500 rpm for 5 min, and the supernatant was collected. The supernatant was taken and the cytokine IL2 was detected according to the instructions of the kit (purchased from Dakewe Biotech Co., Ltd.).

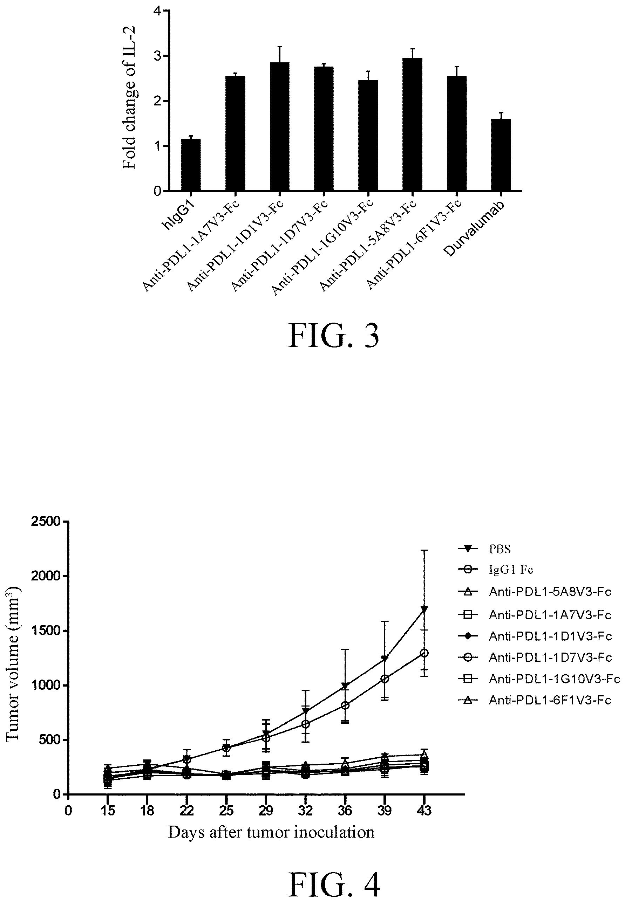

The results are shown in Fig. 3, showing that the anti-PDL1 single-domain antibody fusion protein can enhance the secretion of IL2 by PBMC, and cause a significantly higher fold increase in IL2 than that of the positive control Durvalumab.

### Example 9. Tumor inhibition activity of anti-PDL1-Fc fusion protein

The tumor inhibition activity of anti-PDL1-Fc fusion protein was investigated in an experiment where NOD/SCID mice were subcutaneously transplanted with MC38 cells expressing human PD-L1 (MC38-PDL1) (Nanjing Galaxy Biopharma Co., Ltd) and human peripheral blood mononuclear cells (PBMCs).

The experimental design was as follows: 3 × 10⁶ MC38 cells in 50% Matrigel (purchased from BD) were subcutaneously inoculated into the right flank of 7-8 week-old NOD/SCID mice. 15 days after tumor inoculation, mice with a tumor size of 100-250 mm³ were randomly grouped, and each test group had 4-6 mice. 100 µl of pooled PBMCs (5 × 10⁵) from two healthy donors were injected intratumorally. 3 days after PBMC implantation, anti-PDL1-Fc fusion protein and human IgG1 Fc were administered by subcutaneous injection at a dose of 1 mg/kg, respectively, followed by administration once a week. PBS was injected in the parallel group as a negative control. Determination of tumor volume: The largest long axis (L) and largest wide axis (W) of the tumor were determined by a vernier caliper, and the tumor volume was calculated according to the following formula: V = L × W²/2.

The test results are shown in Fig. 4. The tumor volume of mice inoculated with anti-PDL1-Fc fusion protein was well controlled over time relative to the control group, and shows no significant increase, indicating that anti-PDL1-Fc fusion protein had obvious tumor inhibition effect.

### Example 10. Solubility study of anti-PDL1-Fc fusion proteins

100 mg of purified single-domain antibody was ultrafiltered at 3500 g and 25°C in an ultrafiltration tube (Merck Millipore Ltd.), where the replacement solution was 5 mM phosphate buffer (pH7.2) or 5 mM sodium acetate (pH5.5), the solution was replaced twice, and the concentration was continued until there was no significant change in volume after centrifugation for 20 min. The concentrated solution was centrifuged at 8000 g for 10 min and the protein content was determined. This concentration is the solubility under the described conditions.

**Table 7**

| Protein name | Sequence No. (SEQ ID NO:) | Solubility (mg/ml) |
|---|---|---|
| Anti-PDL 1-1A7V3-Fc | 53 | 128.8 |
| Anti-PDL1-1D1V3-Fc | 54 | 225.3 |
| Anti-PDL 1-1D7V3-Fc | 55 | 132.6 |
| Anti-PDL 1-1G10V3-Fc | 56 | 113.41 |
| Anti-PDL 1-5A8V3-Fc | 57 | 257.11 |
| Anti-PDL 1-6F1V3-Fc | 58 | 59.53 |

According to Table 7, the optimized humanized anti-PDL1-Fc fusion proteins obtained in the present disclosure showed good solubility. As shown in the humanized PDL1 single-domain antibodies in this example, hydrophilic amino acids at positions 44 and 45 of the original FR2 region were substitued into the fairly conserved hydrophobic residues G and L of ordinary human antibodies (Table 4a), with minimal solubility affected.

In summary, the present disclosure effectively overcomes various shortcomings in the prior art, by providing an antibody product with high industrial application value.

A summary of the sequences involved in the present disclosure is shown in Table 8.

**Table8**

| | | |
|---|---|---|
| 1 | FR1 | QLQLVESGGDLVQPGGSLRLSCAAS |
| 2 | FR1 | QLQLVESGGGLVQAGGSLRLSCAAS |
| 3 | FR1 | QVQLVESGGGLVQPGGSLRLSCAAS |
| 4 | FR1 | QVQLVESGGGLVQAGGSLRLSCAAS |
| 5 | FR1 | QLQLVESGGGLVQPGGSLRLSCAAS |
| 6 | CDR1 | GSIFSANL |
| 7 | CDR1 | GGTFITYA |
| 8 | CDR1 | GRAFLTYA |
| 9 | CDR1 | GRPFITYA |
| 10 | CDR1 | GRTFSTHS |
| 11 | FR2 | IDWYRQPPGKQRELVAV |
| 12 | FR2 | MGWFRQAPGKDREFVAG |
| 13 | FR2 | MGWFRQAPGKEREFVAA |
| 14 | FR2 | MHWFRQAPEKERVFVAA |
| 15 | CDR2 | ISSRGIT |
| 16 | CDR2 | ISWSGSST |
| 17 | CDR2 | INWSGSIT |
| 18 | CDR2 | INWNGDST |
| 19 | FR3 | SYADSVKGRFTISRDNAKNTVYLQMNSLKPEDTAVYYC |
| 20 | FR3 | YYTDSVKGRFTISRDNAKNTVYLQMNSLKPGDTAVYYC |
| 21 | FR3 | YYADSVKGRFTISRDNAKNMVYLQMNSLKPEDTAVYYC |
| 22 | FR3 | AYADSVKGRFTISRDNAKNAAYLQMNSLKPDDTAVYYC |
| 23 | FR3 | YYTDSVKGRFTISRDNAQNTVYLQMNSRKPGDTAVYYC |
| 24 | FR3 | YYADSVKGRFTISRDNSKNTVDLQMNNLKPEDTAVYVC |
| 25 | CDR3 | HLFHTDGLGY |
| 26 | CDR3 | AAKISGATREHLMTSYDY |
| 27 | CDR3 | AAKVSGATRDHLMTTYDY |
| 28 | CDR3 | ASKRTAVAPRALNDYDF |
| 29 | CDR3 | AAKISGATREHLRTSYDY |
| 30 | CDR3 | AARPGAPSTIVAMLDY |
| 31 | FR4 | WGQGTQVTVSS |
| 32 | FR4 | GGQGTQVTVSS |
| 33 | 1A7 full-length sequence | QLQLVESGGDLVQPGGSLRLSCAASGSIFSANLIDWYRQPPGKQ RELVAVISSRGITSYADSVKGRFTISRDNAKNTVYLQMNSLKPEDT AVYYCHLFHTDGLGYWGQGTQVTVSS |
| 34 | 1D1 full-length sequence | QLQLVESGGGLVQAGGSLRLSCAASGGTFITYAMGWFRQAPGK DREFVAGISWSGSSTYYTDSVKGRFTISRDNAKNTVYLQMNSLK PGDTAVYYCAAKISGATREHLMTSYDYWGQGTQVTVSS |
| 35 | 1D7 full-length sequence | QVQLVESGGGLVQPGGSLRLSCAASGRAFLTYAMGWFRQAPGK EREFVAAISWSGSSTYYADSVKGRFTISRDNAKNMVYLQMNSLK PEDTAVYYCAAKVSGATRDHLMTTYDYWGQGTQVTVSS |
| 36 | 1G10 full-length sequence | QVQLVESGGGLVQAGGSLRLSCAASGRPFITYAMGWFRQAPGK EREFVAAINWSGSITAYADSVKGRFTISRDNAKNAAYLQMNSLKP DDTAVYYCASKRTAVAPRALNDYDFWGQGTQVTVSS |
| 37 | 5A8 full-length sequence | QLQLVESGGGLVQPGGSLRLSCAASGGTFITYAMGWFRQAPGK DREFVAGISWSGSSTYYTDSVKGRFTISRDNAQNTVYLQMNSRK PGDTAVYYCAAKISGATREHLRTSYDYGGQGTQVTVSS |
| 38 | 6F1 full-length sequence | QLQLVESGGGLVQPGGSLRLSCAASGRTFSTHSMHWFRQAPEK ERVFVAAINWNGDSTYYADSVKGRFTISRDNSKNTVDLQMNNLK PEDTAVYVCAARPGAPSTIVAMLDYWGQGTQVTVSS |
| 39 | hu1A7V3 | QVQLVESGGGLVQPGGSLRLSCAASGSIFSANLIDWYRQAPGKG LELVAVISSRGITSYADSVKGRFTISRDNSKNTLYLQMNSLRAEDT AVYYCHLFHTDGLGYWGQGTLVTVSS |
| 40 | hu1D1V3 | QVQLVESGGGLVQPGGSLRLSCAASGGTFITYAMGWFRQAPGK GLEFVAGISWSGSSTYYADSVKGRFTISRDNSKNTLYLQMNSLRA EDTAVYYCAAKISGATREHLMTSYDYWGQGTLVTVSS |
| 41 | hu1D7V3 | QVQLVESGGGLVQPGGSLRLSCAASGRAFLTYAMGWFRQAPGK GLEFVAAISWSGSSTYYADSVKGRFTISRDNSKNTLYLQMNSLRA EDTAVYYCAAKVSGATRDHLMTTYDYWGQGTLVTVSS |
| 42 | hu1G10V3 | QVQLVESGGGLVQPGGSLRLSCAASGRPFITYAMGWFRQAPGK GLEFVAAINWSGSITAYADSVKGRFTISRDNSKNTLYLQMNSLRAE DTAVYYCASKRTAVAPRALNDYDFWGQGTLVTVSS |
| 43 | hu5A8V3 | QVQLVESGGGLVQPGGSLRLSCAASGGTFITYAMGWFRQAPGK GLEFVAGISWSGSSTYYADSVKGRFTISRDNSKNTLYLQMNSLRA EDTAVYYCAAKISGATREHLRTSYDYWGQGTLVTVSS |
| 44 | hu6F1V3 | QVQLVESGGGLVQPGGSLRLSCAASGRTFSTHSMGWFRQAPGK GLEFVAAINWNGDSTYYADSVKGRFTISRDNSKNTLYLQMNSLRA EDTAVYYCAARPGAPSTIVAMLDYWGQGTLVTVSS |
| 45 | IgG1 FC | EPKSCDKTHTCPPCPAPELLGGPSVFLFPPKPKDTLMISRTPEVT CVVVDVSHEDPEVKFNWYVDGVEVHNAKTKPREEQYNSTYRVV SVLTVLHQDWLNGKEYKCKVSNKALPAPIEKTISKAKGQPREPQV YTLPPSRDELTKNQVSLTCLVKGFYPSDIAVEWESNGQPENNYKT TPPVLDSDGSFFLYSKLTVDKSRWQQGNVFSCSVMHEALHNHYT QKSLSLSPG |
| 46 | IgG4 FC mutant | ESKYGPPCPPCPAPEFLGGPSVFLFPPKPKDTLMISRTPEVTCVV VDVSQEDPEVQFNWYVDGVEVHNAKTKPREEQFNSTYRVVSVL TVLHQDWLNGKEYKCKVSNKGLPSSIEKTISKAKGQPREPQVYT LPPSQEEMTKNQVSLTCLVKGFYPSDIAVEWESNGQPENNYKTT PPVLDSDGSFFLYSRLTVDKSRWQEGNVFSCSVMHEALHNHYT QKSLSLSLG |
| 47 | IgG1 FC mutant | EPKSSDKTHTCPPCPAPELLGGPSVFLFPPKPKDTLMISRTPEVT CVVVAVSHEDPEVKFNWYVDGVEVHNAKTKPREEQYNSTYRVV SVLTVLHQDWLNGKEYKCKVSNKALPAGIEKTISKAKGQPREPQV YTLPPSRDELTKNQVSLTCLVKGFYPSDIAVEWESNGQPENNYKT TPPVLDSDGSFFLYSKLTVDKSRWQQGNVFSCSVMHEALHNHYT QKSLSLSPG |
| 48 | IgG1 FC mutant | EPKSSDKTHTCPPCPAPELLGGPDVFLFPPKPKDTLMISRTPEVT CVVVDVSHEDPEVKFNWYVDGVEVHNAKTKPREEQYNSTYRVV SVLTVLHQDWLNGKEYKCKVSNKALPAPEEKTISKAKGQPREPQ VYTLPPSRDELTKNQVSLTCLVKGFYPSDIAVEWESNGQPENNYK TTPPVLDSDGSFFLYSKLTVDKSRWQQGNVFSCSVMHEALHNHY TQKSLSLSPG |
| 49 | hPDL1-HSA | FTVTVPKDLYVVEYGSNMTIECKFPVEKQLDLAALIVYWEMEDKNI IQFVHGEEDLKVQHSSYRQRARLLKDQLSLGNAALQITDVKLQDA GVYRCMISYGGADYKRITVKVNAPYNKINQRILVVDPVTSEHELTC QAEGYPKAEVIWTSSDHQVLSGKTTTTNSKREEKLFNVTSTLRIN TTTNEIFYCTFRRLDPEENHTAELVIPELPLAHPPNERTGGGGSG GGGDAHKSEVAHRFKDLGEENFKALVLIAFAQYLQQCPFEDHVKL VNEVTEFAKTCVADESAENCDKSLHTLFGDKLCTVATLRETYGEM ADCCAKQEPERNECFLQHKDDNPNLPRLVRPEVDVMCTAFHDN EETFLKKYLYEIARRHPYFYAPELLFFAKRYKAAFTECCQAADKAA CLLPKLDELRDEGKASSAKQRLKCASLQKFGERAFKAWAVARLS QRFPKAEFAEVSKLVTDLTKVHTECCHGDLLECADDRADLAKYIC ENQDSISSKLKECCEKPLLEKSHCIAEVENDEMPADLPSLAADFV ESKDVCKNYAEAKDVFLGMFLYEYARRHPDYSVVLLLRLAKTYET TLEKCCAAADPHECYAKVFDEFKPLVEEPQNLIKQNCELFEQLGE YKFQNALLVRYTKKVPQVSTPTLVEVSRNLGKVGSKCCKHPEAK RMPCAEDYLSVVLNQLCVLHEKTPVSDRVTKCCTESLVNRRPCF SALEVDETYVPKEFNAETFTFHADICTLSEKERQIKKQTALVELVK HKPKATKEQLKAVMDDFAAFVEKCCKADDKETCFAEEGKKLVAA SQAALGL |
| 50 | hPDL1-FC | FTVTVPKDLYVVEYGSNMTIECKFPVEKQLDLAALIVYWEMEDKNI IQFVHGEEDLKVQHSSYRQRARLLKDQLSLGNAALQITDVKLQDA GVYRCMISYGGADYKRITVKVNAPYNKINQRILVVDPVTSEHELTC QAEGYPKAEVIWTSSDHQVLSGKTTTTNSKREEKLFNVTSTLRIN TTTNEIFYCTFRRLDPEENHTAELVIPELPLAHPPNERTDIEGRMD PKSCDKTHTCPPCPAPELLGGPSVFLFPPKPKDTLMISRTPEVTC VVVDVSHEDPEVKFNWYVDGVEVHNAKTKPREEQYASTYRVVS VLTVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPGK |
| 51 | hPD1-FC | LDSPDRPWNPPTFSPALLVVTEGDNATFTCSFSNTSESFVLNWY RMSPSNQTDKLAAFPEDRSQPGQDCRFRVTQLPNGRDFHMSVV RARRNDSGTYLCGAISLAPKAQIKESLRAELRVTERRAEVPTAHP SPSPRPAGQFQDIEGRMDPKSCDKTHTCPPCPAPELLGGPSVFL FPPKPKDTLMISRTPEVTCVVVDVSHEDPEVKFNWYVDGVEVHN AKTKPREEQYASTYRVVSVLTVLHQDWLNGKEYKCKVSNKALPA PIEKTISKAKGQPREPQVYTLPPSRDELTKNQVSLTCLVKGFYPSD IAVEWESNGQPENNYKTTPPVLDSDGSFFLYSKLTVDKSRWQQG NVFSCSVMHEALHNHYTQKSLSLSPGK |
| 52 | mPDL1-HSA | FTITAPKDLYVVEYGSNVTMECRFPVERELDLLALVVYWEKEDEQ VIQFVAGEEDLKPQHSNFRGRASLPKDQLLKGNAALQITDVKLQD AGVYCCIISYGGADYKRITLKVNAPYRKINQRISVDPATSEHELICQ AEGYPEAEVIWTNSDHQPVSGKRSVTTSRTEGMLLNVTSSLRVN ATANDVFYCTFWRSQPGQNHTAELIIPELPATHPPQNRTHGGGG SGGGGDAHKSEVAHRFKDLGEENFKALVLIAFAQYLQQCPFEDH VKLVNEVTEFAKTCVADESAENCDKSLHTLFGDKLCTVATLRETY GEMADCCAKQEPERNECFLQHKDDNPNLPRLVRPEVDVMCTAF HDNEETFLKKYLYEIARRHPYFYAPELLFFAKRYKAAFTECCQAAD KAACLLPKLDELRDEGKASSAKQRLKCASLQKFGERAFKAWAVA RLSQRFPKAEFAEVSKLVTDLTKVHTECCHGDLLECADDRADLAK YICENQDSISSKLKECCEKPLLEKSHCIAEVENDEMPADLPSLAAD FVESKDVCKNYAEAKDVFLGMFLYEYARRHPDYSVVLLLRLAKTY ETTLEKCCAAADPHECYAKVFDEFKPLVEEPQNLIKQNCELFEQL GEYKFQNALLVRYTKKVPQVSTPTLVEVSRNLGKVGSKCCKHPE AKRMPCAEDYLSVVLNQLCVLHEKTPVSDRVTKCCTESLVNRRP CFSALEVDETYVPKEFNAETFTFHADICTLSEKERQIKKQTALVEL VKHKPKATKEQLKAVMDDFAAFVEKCCKADDKETCFAEEGKKLV AASQAALGL |
| 53 | Anti-PDL 1-1A7V3-Fc | QVQLVESGGGLVQPGGSLRLSCAASGSIFSANLIDWYRQAPGKG LELVAVISSRGITSYADSVKGRFTISRDNSKNTLYLQMNSLRAEDT AVYYCHLFHTDGLGYWGQGTLVTVSSEPKSCDKTHTCPPCPAPE LLGGPSVFLFPPKPKDTLMISRTPEVTCVVVDVSHEDPEVKFNWY VDGVEVHNAKTKPREEQYNSTYRVVSVLTVLHQDWLNGKEYKC KVSNKALPAPIEKTISKAKGQPREPQVYTLPPSRDELTKNQVSLTC LVKGFYPSDIAVEWESNGQPENNYKTTPPVLDSDGSFFLYSKLTV DKSRWQQGNVFSCSVMHEALHNHYTQKSLSLSPG |
| 54 | Anti-PDL 1-1D1V3-Fc | QVQLVESGGGLVQPGGSLRLSCAASGGTFITYAMGWFRQAPGK GLEFVAGISWSGSSTYYADSVKGRFTISRDNSKNTLYLQMNSLRA EDTAVYYCAAKISGATREHLMTSYDYWGQGTLVTVSSEPKSCDK THTCPPCPAPELLGGPSVFLFPPKPKDTLMISRTPEVTCVVVDVS HEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PG |
| 55 | Anti-PDL1-1D7V3-Fc | QVQLVESGGGLVQPGGSLRLSCAASGRAFLTYAMGWFRQAPGK GLEFVAAISWSGSSTYYADSVKGRFTISRDNSKNTLYLQMNSLRA EDTAVYYCAAKVSGATRDHLMTTYDYWGQGTLVTVSSEPKSCDK THTCPPCPAPELLGGPSVFLFPPKPKDTLMISRTPEVTCVVVDVS HEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PG |
| 56 | Anti-PDL 1-1G10V3-Fc | QVQLVESGGGLVQPGGSLRLSCAASGRPFITYAMGWFRQAPGK GLEFVAAINWSGSITAYADSVKGRFTISRDNSKNTLYLQMNSLRAE DTAVYYCASKRTAVAPRALNDYDFWGQGTLVTVSSEPKSCDKTH TCPPCPAPELLGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALPAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG |
| 57 | Anti-PDL 1-5A8V3-Fc | QVQLVESGGGLVQPGGSLRLSCAASGGTFITYAMGWFRQAPGK GLEFVAGISWSGSSTYYADSVKGRFTISRDNSKNTLYLQMNSLRA EDTAVYYCAAKISGATREHLRTSYDYWGQGTLVTVSSEPKSCDKT HTCPPCPAPELLGGPSVFLFPPKPKDTLMISRTPEVTCVVVDVSH EDPEVKFNWYVDGVEVHNAKTKPREEQYNSTYRVVSVLTVLHQ DWLNGKEYKCKVSNKALPAPIEKTISKAKGQPREPQVYTLPPSRD ELTKNQVSLTCLVKGFYPSDIAVEWESNGQPENNYKTTPPVLDSD GSFFLYSKLTVDKSRWQQGNVFSCSVMHEALHNHYTQKSLSLSP G |
| 58 | Anti-PDL 1-6F1V3-Fc | QVQLVESGGGLVQPGGSLRLSCAASGRTFSTHSMGWFRQAPGK GLEFVAAINWNGDSTYYADSVKGRFTISRDNSKNTLYLQMNSLRA EDTAVYYCAARPGAPSTIVAMLDYWGQGTLVTVSSEPKSCDKTH TCPPCPAPELLGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALPAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG |
| 59 | humanized FR2 | IDWYRQAPGKGLELVAV |
| 60 | humanized FR2 | MGWFRQAPGKGLEFVAG |
| 61 | humanized FR2 | MGWFRQAPGKGLEFVAA |
| 62 | humanized FR3 | SYADSVKGRFTISRDNSKNTLYLQMNSLRAEDTAVYYC |
| 63 | humanized FR3 | YYADSVKGRFTISRDNSKNTLYLQMNSLRAEDTAVYYC |
| 64 | humanized FR3 | AYADSVKGRFTISRDNSKNTLYLQMNSLRAEDTAVYYC |
| 65 | humanized FR4 | WGQGTLVTVSS |
| 66 | PDL1-56 dAb | AADSFEDPTCTLVTSSGAFQY |
| 67 | InnoventNb | AAPKVGLGPRTALGHLAFMTLPALNY |
| 68 | Nb 43 | AADDDYYAFLSRGARDFRY |

The above embodiments only exemplarily illustrate the principles and effects of the present disclosure, but are not used to limit the present disclosure. Any person skilled in the art may make modifications or changes on the foregoing embodiments without departing from the spirit and scope of the present disclosure. Therefore, all equivalent modifications or changes made by a person of ordinary skill in the art without departing from the spirit and technical idea of the present disclosure shall be covered by the claims of the present disclosure.

## Claims

1. An anti-PD-L1 single-domain antibody, having a complementarity determining region (CDR) comprising CDR1-CDR3 having amino acid sequences shown below:
(1) CDR1 as shown in SEQ ID NO: 6, CDR2 as shown in SEQ ID NO: 15, and CDR3 as shown in SEQ ID NO: 25; or
(2) CDR1 as shown in SEQ ID NO: 7, CDR2 as shown in SEQ ID NO: 16, and CDR3 as shown in SEQ ID NO: 26; or
(3) CDR1 as shown in SEQ ID NO: 8, CDR2 as shown in SEQ ID NO: 16, and CDR3 as shown in SEQ ID NO: 27; or
(4) CDR1 as shown in SEQ ID NO: 9, CDR2 as shown in SEQ ID NO: 17, and CDR3 as shown in SEQ ID NO: 28; or
(5) CDR1 as shown in SEQ ID NO: 7, CDR2 as shown in SEQ ID NO: 16, and CDR3 as shown in SEQ ID NO: 29; or
(6) CDR1 as shown in SEQ ID NO: 10, CDR2 as shown in SEQ ID NO: 18, and CDR3 as shown in SEQ ID NO: 30.

2. The single-domain antibody according to claim 1, wherein the anti-PD-L1 single-domain antibody further comprises framework regions (FR), comprising FR1-FR4 having amino acid sequences shown below:
(1') FR1 as shown in SEQ ID NO: 1, FR2 as shown in SEQ ID NO: 11, FR3 as shown in SEQ ID NO: 19, and FR4 as shown in SEQ ID NO: 31; or
(2') FR1 as shown in SEQ ID NO: 2, FR2 as shown in SEQ ID NO: 12, FR3 as shown in SEQ ID NO: 20, and FR4 as shown in SEQ ID NO: 31; or
(3') FR1 as shown in SEQ ID NO: 3, FR2 as shown in SEQ ID NO: 13, FR3 as shown in SEQ ID NO: 21, and FR4 as shown in SEQ ID NO: 31; or
(4') FR1 as shown in SEQ ID NO: 4, FR2 as shown in SEQ ID NO: 13, FR3 as shown in SEQ ID NO: 22, and FR4 as shown in SEQ ID NO: 31; or
(5') FR1 as shown in SEQ ID NO: 5, FR2 as shown in SEQ ID NO: 12, FR3 as shown in SEQ ID NO: 23, and FR4 as shown in SEQ ID NO: 32; or
(6') FR1 as shown in SEQ ID NO: 5, FR2 as shown in SEQ ID NO: 14, FR3 as shown in SEQ ID NO: 24, and FR4 as shown in SEQ ID NO: 31.

3. The single-domain antibody according to claim 1 or 2, wherein the anti-PD-L1 single-domain antibody comprises:
(a) a single-domain antibody having an amino acid sequence as shown in any one of SEQ ID NOs: 33-38; or
(b) a single-domain antibody having an amino acid sequence that has sequence identity of 80% or above to the sequence as shown in any one of SEQ ID NOs: 33-38, and having functions of the (a) single-domain antibody.

4. The single-domain antibody according to claim 1, wherein the anti-PD-L1 single-domain antibody is a humanized antibody, wherein preferably, a framework region (FR) of the single-domain antibody comprises FR1-FR4 having amino acid sequences selected from the group consisting of:
FR1 as shown in SEQ ID NO: 3;
FR2 as shown in SEQ ID NO: 59-61;
FR3 as shown in SEQ ID NO: 62-64; and
FR4 as shown in SEQ ID NO: 65.

5. The single-domain antibody according to claim 1 or 4, wherein the anti-PD-L1 single-domain antibody is a humanized antibody, and comprises:
(i) a single-domain antibody having an amino acid sequence as shown in SEQ ID NO: 39-44; or
(ii) a single-domain antibody having an amino acid sequence that has sequence identity of 80% or above to the sequence as shown in any one of SEQ ID NOs: 39-44, and having functions of the (i) single-domain antibody.

6. A fusion protein, comprising:
a first domain, which is a single-domain antibody as in any one of claims 1 to 4; and
a second domain, having an effect of extending the half-life and/or binding to effector cells.

7. The fusion protein according to claim 6, wherein the second domain comprises:
an immunoglobulin Fc region, preferably a human immunoglobulin Fc region; and/or
serum albumin or a fragment thereof, a domain that binds to serum albumin, polyethylene glycol, a polyethylene glycol-liposome complex, or a combination thereof; and/or
a molecule that has an affinity for surface molecules of T cells and/or are capable of binding to a surface molecule present on T cells, wherein preferably, the surface molecule comprises CD3.

8. The fusion protein according to claim 7, wherein the human immunoglobulin Fc region comprises a mutation for altering an Fc-mediated effector function, wherein the effector function comprises CDC activity, ADCC activity, ADCP activity, or a combination thereof; or
the immunoglobulin is selected from IgG, IgA1, IgA2, IgD, IgE, IgM, or a combination thereof; and the IgG is selected from IgG1, IgG2, IgG3, or IgG4 subtype, or a combination thereof; or
the amino acid sequence of the immunoglobulin Fc region is one selected from SEQ ID NOs: 45-48; or
a linking peptide is provided between the first domain and the second domain; and the linking peptide is preferably selected from a flexible polypeptide chain consisting of alanine and/or serine and/or glycine, and has a length of preferably 3-30 amino acids.

9. An isolated polynucleotide, encoding a single-domain antibody according to any one of claims 1 to 5; or encoding a fusion protein according to any one of claims 6 to 8.

10. A construct, comprising an isolated polynucleotide according to claim 9.

11. An antibody expressing system, comprising a construct according to claim 10 or having a genome integrated with an exogenous polynucleotide according to claim 9, wherein preferably, the expressing system is a cell expression system.

12. A method for producing a single-domain antibody according to any one of claims 1 to 5, or a fusion protein according to any one of claims 6 to 8, comprising: under conditions suitable for expressing the antibody, expressing the antibody or fusion protein using the antibody expressing system according to claim 11; and preferably, the method further comprises purifying and isolating the antibody or fusion protein.

13. An immunoconjugate, comprising:
(A) a single-domain antibody according to any one of claims 1 to 5 or a fusion protein according to any one of claims 5 to 7, and
(B) a functional molecule linked to (A).

14. The immunoconjugate according to claim 12, wherein (B) comprises
a cytotoxin, a radioisotope, a biologically active protein, a molecule targeting a tumor surface marker, a tumor-suppressing molecule, a molecule or detectable marker that targets a surface marker of an immune cell, or an extracellular hinge region, a transmembrane region and intracellular signaling region based on chimeric antigen receptor technology, or a combination thereof; preferably, the molecule targeting the tumor surface marker is an antibody or ligand that binds to the tumor surface marker; or the tumor-suppressing molecule is an anti-tumor cytokine or anti-tumor toxin.

15. A pharmaceutical composition, comprising a single-domain antibody according to any one of claims 1 to 5, a fusion protein according to any one of claims 6 to 7, or an immunoconjugate according to any one of claims 13 to 14.

16. The pharmaceutical composition according to claim 15, further comprising a pharmaceutically acceptable carrier.

17. The use of a single-domain antibody according to any one of claims 1 to 5, a fusion protein according to any one of claims 6 to 8, an immunoconjugate according to any one of claims 13 to 14, or a pharmaceutical composition according to any one of claims 15 to 16 in the preparation of formulations, kits, or pharmaceutical packs for the diagnosis, treatment or prevention of cancers.

18. The use according to claim 17, wherein the cancers comprise lung cancer, melanoma, gastric cancer, ovarian cancer, colon cancer, liver cancer, kidney cancer, bladder cancer, breast cancer, classic Hodgkin lymphoma, hematological malignancies, head and neck cancer or nasopharyngeal cancer, or a combination thereof.

19. The use of the single-domain antibody according to any one of claims 1 to 5, the fusion protein according to any one of claims 6 to 8, the immunoconjugate according to any one of claims 13 to 14, or the pharmaceutical composition according to any one of claims 15 to 16 in preparation of formulations, kits, or pharmaceutical packs for the treatment or prevention of infectious diseases or chronic inflammatory diseases.

20. A kit or pharmaceutical pack, comprising a single-domain antibody according to any one of claims 1 to 5, a fusion protein according to any one of claims 6 to 8, an immunoconjugate according to any one of claims 13 to 14, or a pharmaceutical composition according to any one of claims 15 to 16.
